(19) Europäisches Patentamt
European Patent Office
Office européen des brevets

(11) Publication number: **0 190 709 B1**

# (12) EUROPEAN PATENT SPECIFICATION

(45) Date of publication of patent specification: **06.12.89**

(21) Application number: **86101392.8**

(22) Date of filing: **04.02.86**

(51) Int. Cl.[4]: **C 07 D 498/18,**
C 07 D 519/00, A 61 K 31/535
// (C07D498/18, 307:00, 267:00, 265:00),(C07D519/00, 498:00, 491:00)

## (54) Benzoxazinorifamycin derivative, process for preparing the same and anti-bacterial agent containing the same.

(30) Priority: **05.02.85 JP 20384/85**

(43) Date of publication of application:
**13.08.86 Bulletin 86/33**

(45) Publication of the grant of the patent:
**06.12.89 Bulletin 89/49**

(84) Designated Contracting States:
**BE CH DE FR GB IT LI NL SE**

(56) References cited:
**GB-A-1 081 757**

(73) Proprietor: **KANEGAFUCHI KAGAKU KOGYO KABUSHIKI KAISHA**
**2-4 Nakanoshima 3-chome**
**Kita-ku Osaka-shi Osaka-fu (JP)**

(72) Inventor: **Yamane, Takehiko**
**5-1-1121, Oakashi-cho 2-chome**
**Akashi-shi Hyogo-ken (JP)**
Inventor: **Hashizume, Takuji**
**2-63, Okihama-cho Takasago-cho**
**Takasago-shi Hyogo-ken (JP)**
Inventor: **Yamashita, Katsuji**
**14-10, Takakuradai 8-chome Suma-ku**
**Kobe-shi Hyogo-ken (JP)**
Inventor: **Watanabe, Kiyoshi**
**15-41, Matsugaoka 5-chome**
**Akashi-shi Hyogo-ken (JP)**

(74) Representative: **Türk, Gille, Hrabal**
**Bruckner Strasse 20**
**D-4000 Düsseldorf 13 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 190 709 B1

## Description

The present invention relates to a novel rifamycin derivative or salts thereof, a process for preparing the same and antibacterial agents containing the same as an effective component. More particularly, the present invention relates to a novel rifamycin derivative having the general formula (I):

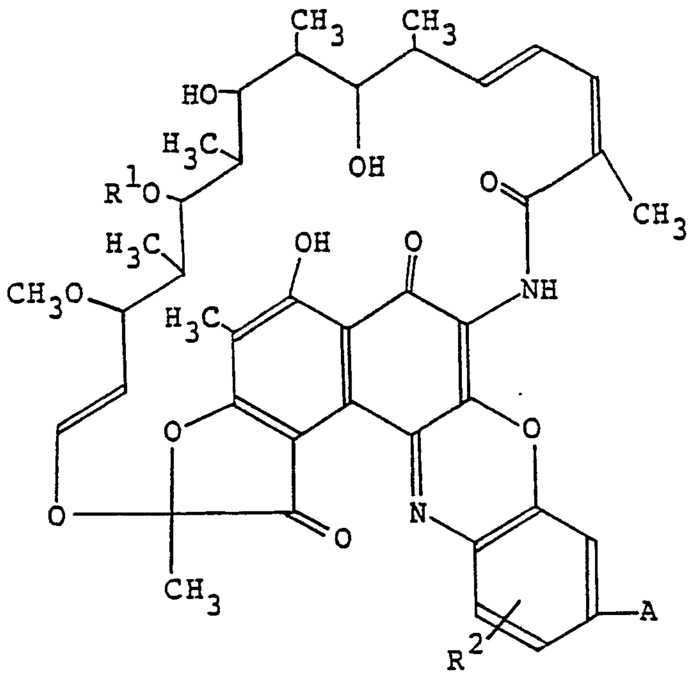

(I)

wherein $R^1$ is hydrogen atom or acetyl group; $R^2$ is hydrogen atom or hydroxyl group; A is a group represented by the formula:

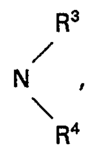
,

wherein $R^3$ is an alkyl group with 1 to 5 carbon atoms or an alkoxyalkyl group with 2 to 6 carbon atoms and $R^4$ is an alkyl group with 1 to 5 carbon atoms, a group represented by the formula: $—(CH_2)_aX^1$, wherein a is 1 to 4 and $X^1$ is ethynyl group, cyano group, a group having the formula:

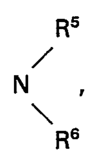
,

wherein $R^5$ and $R^6$ are the same or different from each other and are hydrogen atom or an alkyl group with 1 to 3 carbon atoms, a group represented by the formula: $OR^7$, wherein $R^7$ is hydrogen atom or an alkyl group with 1 to 4 carbon atoms, a group represented by the formula:

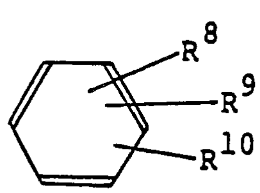
,

wherein $R^8$, $R^9$ and $R^{10}$ are the same or different from each other and are hydrogen atom or an alkoxy group with 1 to 3 carbon atoms, or a group represented by the formula:

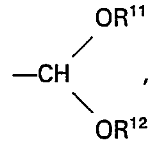 ,

wherein $R^{11}$ and $R^{12}$ are the same or different from each other and are an alkyl group with 1 to 3 carbon atoms; a cycloalkyl group with 3 to 8 carbon atoms, a group represented by the formula:

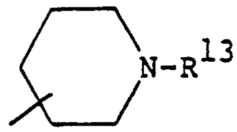 ,

wherein $R^{13}$ is hydrogen atom or an alkyl group with 1 to 3 carbon atoms, or a group represented by the formula: $-CH_2(CHOH)_4CH_2OH$; a group represented by the formula:

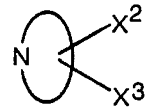 ,

wherein

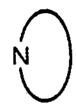

is a 3 to 10 membered cyclic amino group with 2 to 9 carbon atoms, $X^2$ is hydrogen atom or an alkyl group having 1 to 4 carbon atoms and $X^3$ is hydrogen atom, an alkyl group with 1 to 3 carbon atoms, hydroxyl group, a group represented by the formula:

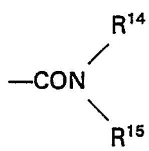 ,

wherein $R^{14}$ and $R^{15}$ are the same or different from each other and are hydrogen atom or an alkyl group with 1 to 4 carbon atoms, a hydroxyalkyl group with 1 to 3 carbon atoms or cyclic or noncyclic amino group with 2 to 6 carbon atoms, or $X^2$ and $X^3$ when taken together represent $=O$ group or the group represented by the formula: $-O(CH_2)_bO-$, wherein b is 2 to 4, a group represented by the formula:

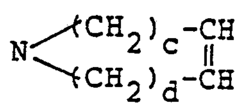 ,

wherein c and d are the same or different from each other and are 1 to 4, a group represented by the formula:

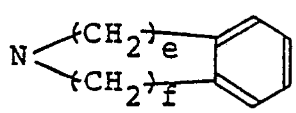 ,

wherein e and f are the same or different from each other and are 1 to 4, or a group represented by the formula:

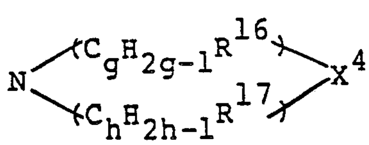 ,

wherein g and h are the same or different from each other and are 1 to 4, $R^{16}$ and $R^{17}$ are the same or different from each other and are hydrogen atom or an alkyl group with 1 to 3 carbon atoms and $X^4$ is oxygen atom, sulfur atom or a group represented by the formula: $NR^{18}$, wherein $R^{18}$ is hydrogen atom, an alkyl group with 1 to 3 carbon atoms, a phenyl group represented by the formula:

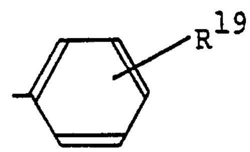

wherein $R^{19}$ is hydrogen atom or trifluoromethyl group, or a group represented by the formula:

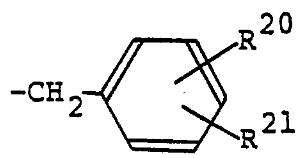

wherein $R^{20}$ and $R^{21}$ are hydrogen atoms or when taken together represent the group having the formula: $-OCH_2O-$, or salts thereof, a process for preparing the same and antibacterial agents containing the same as an effective component.

The rifamycin derivative of the present invention is a novel compound which has not yet been reported in the literature.

GB—A—1 081 757 discloses derivatives of rifamycin O and S and the antibiotic activity thereof. The absorption into blood of these compounds after oral administration is only low, therefore high doses of the compounds have to be administered.

According to the present invention, there are provided a rifamycin derivative having the general formula (I):

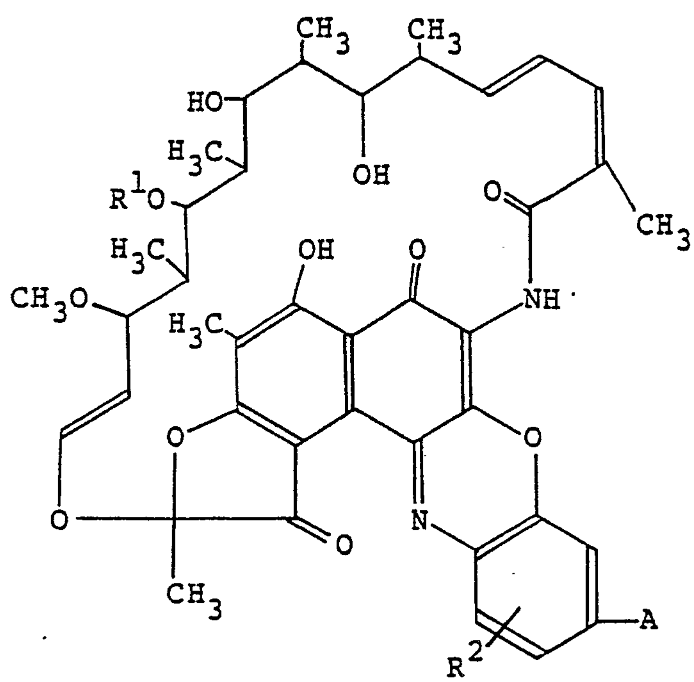

(I)

wherein $R^1$ is hydrogen atom or acetyl group; $R^2$ is hydrogen atom or hydroxyl group; A is a group represented by the formula:

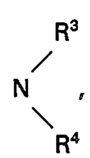

wherein $R^3$ is an alkyl group with 1 to 5 carbon atoms or an alkoxyalkyl group with 2 to 6 carbon atoms and $R^4$ is an alkyl group with 1 to 5 carbon atoms, a group represented by the formula: $-(CH_2)_aX^1$, wherein a is 1 to 4 and $X^1$ is ethynyl group, cyano group, a group having the formula:

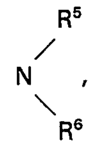
,

wherein $R^5$ and $R^6$ are the same or different from each other and are hydrogen atom or an alkyl group with 1 to 3 carbon atoms, a group represented by the formula: $OR^7$, wherein $R^7$ is hydrogen atom or an alkyl group with 1 to 4 carbon atoms, a group represented by the formula:

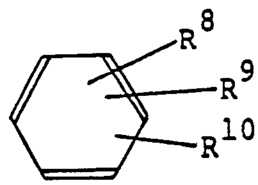
,

wherein $R^8$, $R^9$ and $R^{10}$ are the same or different from each other and are hydrogen atom or an alkoxy group with 1 to 3 carbon atoms, or a group represented by the formula:

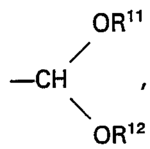
,

wherein $R^{11}$ and $R^{12}$ are the same or different from each other and are an alkyl group with 1 to 3 carbon atoms; a cycloalkyl group with 3 to 8 carbon atoms, a group represented by the formula:

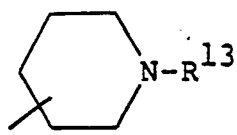
,

wherein $R^{13}$ is hydrogen atom or an alkyl group with 1 to 3 carbon atoms, or a group represented by the formula: $-CH_2(CHOH)_4CH_2OH$; a group represented by the formula:

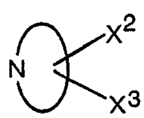
,

wherein

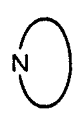

is a 3 to 10 membered cyclic amino group with 2 to 9 carbon atoms, $X^2$ is hydrogen atom or an alkyl group having 1 to 4 carbon atoms and $X^3$ is hydrogen atom, an alkyl group with 1 to 3 carbon atoms, hydroxyl group, a group represented by the formula:

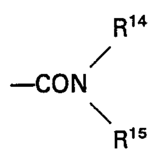
,

wherein $R^{14}$ and $R^{15}$ are the same or different from each other and are hydrogen atom or an alkyl group with

1 to 4 carbon atoms, a hydroxyalkyl group with 1 to 3 carbon atoms or cyclic or noncyclic amino group with 2 to 6 carbon atoms, or $X^2$ and $X^3$ when taken together represent = O group or the group represented by the formula: $—O(CH_2)_bO—$, wherein b is 2 to 4, a group represented by the formula:

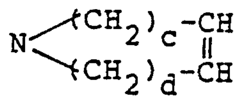,

wherein c and d are the same or different from each other and are 1 to 4, a group represented by the formula:

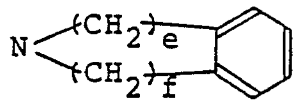,

wherein e and f are the same or different from each other and are 1 to 4, or a group represented by the formula:

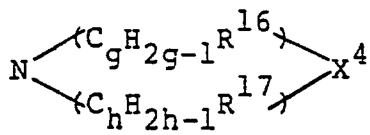,

wherein g and h are the same or different from each other and are 1 to 4, $R^{16}$ and $R^{17}$ are the same or different from each other and are hydrogen atom or an alkyl group with 1 to 3 carbon atoms and $X^4$ is oxygen atom, sulfur atom or a group represented by the formula: $NR^{18}$, wherein $R^{18}$ is hydrogen atom, an alkyl group with 1 to 3 carbon atoms, a phenyl group represented by the formula:

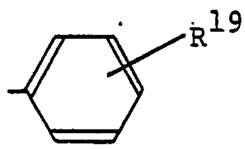,

wherein $R^{19}$ is hydrogen atom or trifluoromethyl group, or a group represented by the formula:

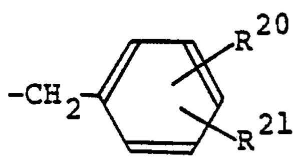,

wherein $R^{20}$ and $R^{21}$ are hydrogen atoms or when taken together represent the group having the formula: $—OCH_2O—$, or salts thereof, a process for preparing the same and antibacterial agents containing the same as an effective component.

As the result of the present inventors study, it was found that a rifamycin derivative having the general formula (I):

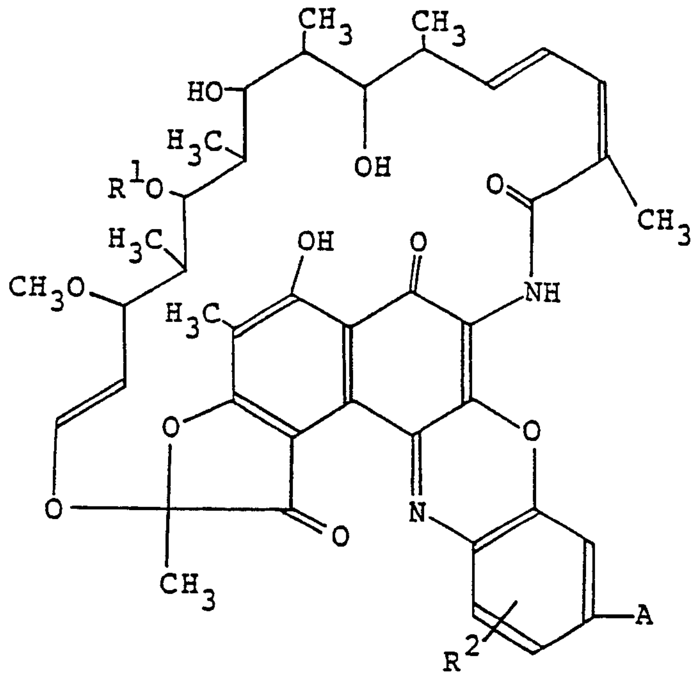

(I)

wherein $R^1$, $R^2$ and A are as defined above, could be prepared by reacting a rifamycin derivative having the general formula (II):

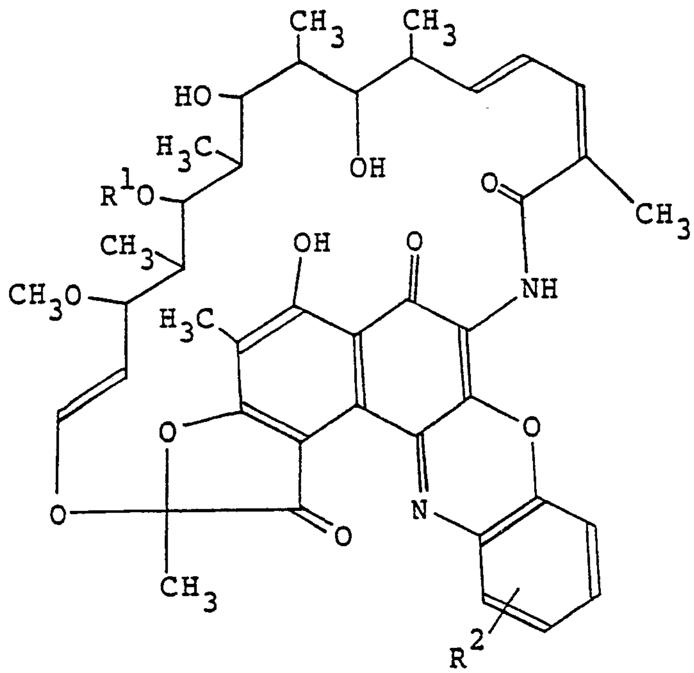

(II)

wherein $R^1$ and $R^2$ are as defined above, with an amine having the general formula: AH, wherein A is as defined above and the obtained rifamycin derivative having the general formula (I) possesses a high degree of activity against a large number of microorganisms.

The rifamycin derivative having the general formula (I) is soluble in various kinds of organic solvents, e.g. halogenated hydrocarbons such as chloroform, alcohols such as ethanol, esters such as ethyl acetate, aromatic hydrocarbons such as benzene and ethers such as tetrahydrofuran.

The rifamycin derivative of the present invention having the general formula (I) can form a salt with both base and acid. Any base or acid which can form a salt with the rifamycin derivative having the general formula (I) may be employed. Examples of the salt with base are metallic salt, especially alkali metal salt or alkaline earth metal salt, ammonium salt and amine salts, especialy a salt with methylamine, ethylamine, diethylamine, triethylamine, pyrrolidine, morpholine or hexamethyleneimine.

Examples of the salt with acid are a salt with mineral acid such as sulfuric acid or hydrochloric acid and a salt with organic acid such as p-toluenesulfonic acid, trifluoroacetic acid or acetic acid.

The rifamycin derivative of the present invention having the general formula (I) can be prepared by reacting the rifamycin derivative having the general formula (II) dissolved in an organic solvent such as methanol, ethanol, tetrahydrofuran, N,N-dimethylformamide or dimethylsulfoxide with the amine having the general formula: AH wherein A is as defined above, in the presence or absence of acid such as hydrochloric acid at a temperature of from −20°C to a boiling point of the solvent for 1 hour to 1 month and in the presence or absence of oxidizing agents.

Examples of the reaction solvent employed in the present invention are, for instance, methanol, ethanol, isopropyl alcohol, tetrahydrofuran, pyridine, acetone, ethyl acetate, chloroform, N,N-dimethylformamide, dimethylsulfoxide, and the like. Among them, pyridine, N,N-dimethylformamide or dimethylsulfoxide is preferably employed in the present invention with an excellent result.

The reaction is carried out at a temperature ranging from −20°C to a boiling point of the solvent, preferably from −5° to 50°C.

Though the reaction may be carried out for around 1 hour to around 1 month, the optimum reaction time should be determined by following the proceeding of the reaction by thin layer chromatography and the like since it varies depending on a kind and an amount of the amine employed in the present invention, the presence or absence of oxidizing agent, a kind and an amount thereof, when present, the reaction temperature and the like.

When the reaction is carried out in the presence of the oxidizing agent, air, oxygen, manganese dioxide, lead dioxide, silver oxide, potassium ferricyanide, hydrogen peroxide and the like are employed as the oxidizing agent. Among them, manganese dioxide, silver oxide or potassium ferricyanide is preferably employed in the present invention with an excellent result.

The rifamycin derivative having the general formula (I), wherein $R^1$ is hydrogen atom and $R^2$ and A are as defined above, can also be prepared by hydrolyzing the rifamycin derivative having the general formula (I), wherein $R^1$ is acetyl group and $R^2$ and A are as defined above, with acid or base. Examples of acid employed for hydrolysis are, for instance, a mineral acid such as sulfuric acid or hydrochloric acid and an organic acid such as *p*-toluenesulfonic acid or trifluoroacetic acid. Examples of base employed for hydrolysis are, for instance, alkali metal hydroxide such as sodium hydroxide or pòtassium hydroxide, alkaline earth metal hydroxide such as calcium hydroxide or barium hydroide, and an organic base such as 1,5-diazabicyclo[4.3.0]non-5-ene or 1,8-diazabicyclo[5.4.0]undec-7-ene.

Preferably the hydrolysis reaction is carried out at room temperature employing alkali metal hydroxide such as sodium hydroxcide or potassium hydroxide and a solvent such as water-containing methanol or water-containing pyridine.

The rifamycin derivative of the present invention having the general formula (I), which is a dark purple solid, can be separated and purified from the reaction products in a relatively easy manner, i.e. an excess amount of the amine having the formula: AH and the reaction solvent are removed from the reaction system to give a crude product, which is then purified by crystallization, column-chromatography and the like.

The rifamycin derivative of the present invention having the general formula (I) can also be converted to the rifamycin derivative having the general formula (III):

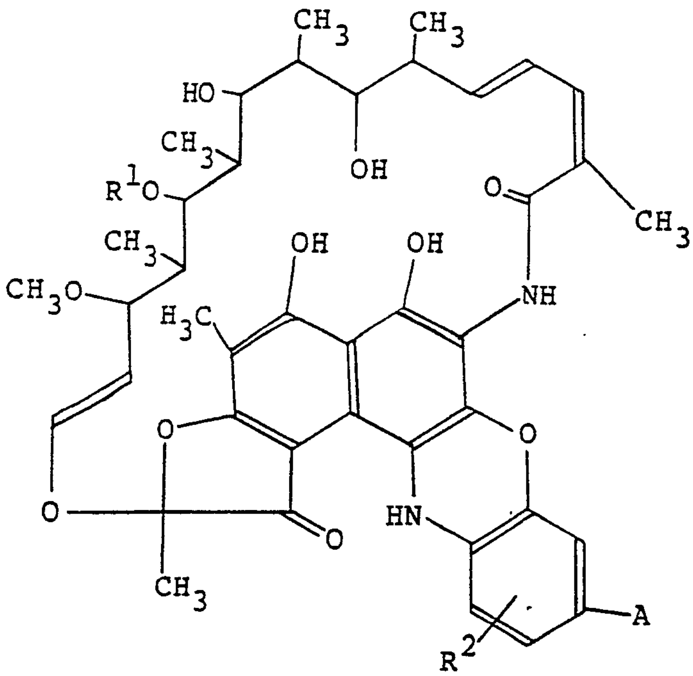

(III)

wherein $R^1$, $R^2$ and A are as defined above, by reducing the rifamycin derivative having the general formula (I) with reducing agent such as ascorbic acid, sodium hydrosulfite or the like.

Typical examples of the rifamycin derivative of the present invention having the general formula (I) are shown in Table 1.

Table 1

| Derivative No. | $R^1$ | $R^2$ | A | | Crystal form | Thin layer chromatography | | Infrared absorption spectrum ($cm^{-1}$) | Chemical shift of nuclear magnetic resonance spectrum derived from the amine induced (δ, ppm**) |
|---|---|---|---|---|---|---|---|---|---|
| | | | $R^3$ | $R^4$ | | Rf | Solvent system* | | |
| 1 | $COCH_3$ | H | $CH_3$ | $CH_3$ | flake | 0.26 | B | 1600 (C=0) | 3.16 ($CH_3$, 6H, s) |
| 2 | $COCH_3$ | H | $CH_3$ | $C_2H_5$ | flake | 0.33 | B | 1600 (C=0) | 1.27 ($CH_3$, 3H, t), 3.13 ($CH_3$, 3H, s) and 3.56 ($CH_2$, 2H, q) |
| 3 | $COCH_3$ | H | $C_2H_5$ | $C_2H_5$ | flake | 0.39 | B | 1600 (C=0) | 1.28 ($CH_3$, 6H, s) and 3.54 ($CH_2$, 4H, q) |
| 4 | $COCH_3$ | H | $C_3H_7$ | $C_3H_7$ | flake | 0.43 | B | 1601 (C=0) | 1.00 ($CH_3$, 6H, t) and 3.37 —$NCH_2$—, 4H, br) |
| 5 | $COCH_3$ | H | $CH_3$ | $CH_2C$ CH | flake | 0..36 | B | 1604 (C=0) | 3.15 ($CH_3$, 3H, s) |

- continued -

- continued -

| Derivative No. | $R^1$ | $R^2$ | A | | Crystal form | Thin layer chromatography | | Infrared absorption spectrum ($cm^{-1}$) | Chemical shift of nuclear magnetic resonance spectrum derived from the amine induced (δ, ppm**) |
|---|---|---|---|---|---|---|---|---|---|
| | | | $R^3$ | $R^4$ | | Rf | Solvent system* | | |
| 6 | $COCH_3$ | H | $CH_3$ | $CH_2CH_2CN$ | needle | 0.31 | C | 1601 (C=0) | 2.73 ($CH_2$, 2H, br) and 3.27 ($CH_3$, 3H, s) |
| 7 | $COCH_3$ | H | $CH_3$ | $CH_2CH_2N(CH_3)_2$ | amorphous | 0.34 | D | 1600 (C=0) | 2.33 ($N(CH_3)_2$, 6H, s) and 3.15 (N—$CH_3$, 3H, s) |
| 8 | $COCH_3$ | H | $CH_3$ | $CH_2CH_2OH$ | amorphous | 0.43 | D | 1606 (C=0) | 3.20 ($CH_3$, 3H, s) and 3.73 ($CH_2CH_2$, 4H, d) |
| 9 | $COCH_3$ | H | $CH_2CH_2OC_2H_5$ | $CH_2CH_2OC_2H_5$ | needle | 0.32 | B | 1601 (C=0) | 1.20 ($CH_3$, 6H, t), 3.47 (—$OCH_2CH_3$, 4H, q) and 3.67 ($NCH_2CH_2O$, 8H, br) |

- continued -

- continued -

| Derivative No. | $R^1$ | $R^2$ | A: $R^3$ | A: $R^4$ | Crystal form | Thin layer chromatography: Rf | Thin layer chromatography: Solvent system* | Infrared absorption spectrum ($cm^{-1}$) | Chemical shift of nuclear magnetic resonance spectrum derived from the amine induced (δ, ppm**) |
|---|---|---|---|---|---|---|---|---|---|
| 10 | $COCH_3$ | H | $CH_2CH_2CH_2OCH_3$ | 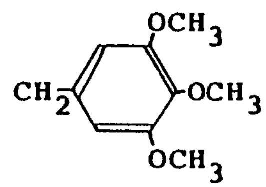 | amorphous | 0.37 | B | 1598 (C=0) | 3.35 ($CH_2OCH_3$, 3H, s), 3.80, ( 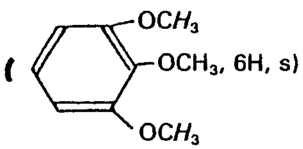 , 6H, s) 3.83 ( 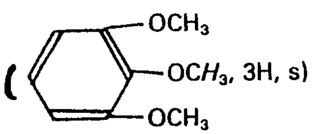 , 3H, s) 4.67 ($CH_2$- 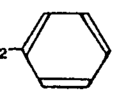 , 2H, s) |
| 11 | $COCH_3$ | H | $CH_3$ | $CH_2CH(OCH_3)_2$ | amorphous | 0.29 | B | 1600 (C=0) | 3.20 ($CH_3$, 3H, s) and 3.43 ($OCH_3$, 6H, s) |

- continued -

- continued -

| Derivative No. | $R^1$ | $R^2$ | A: $R^3$ | A: $R^4$ | Crystal form | Thin layer chromatography: Rf | Thin layer chromatography: Solvent system* | Infrared absorption spectrum ($cm^{-1}$) | Chemical shift of nuclear magnetic resonance spectrum derived from the amine induced (δ, ppm**) |
|---|---|---|---|---|---|---|---|---|---|
| 12 | $COCH_3$ | H | $CH_3$ | 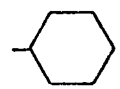 | amorphous | 0.40 | B | 1602 (C=0) | 3.06 ($CH_3$, 3H, s) |
| 13 | $COCH_3$ | H | $CH_3$ | 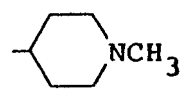 | flake | 0.27 | D | 1602 (C=0) | 3.03 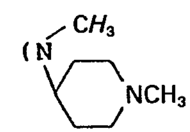 3H, s) |
| 14 | $COCH_3$ | H | $CH_3$ | $CH_2(CHOH)_4CH_2OH$ | amorphous | 0.13 | D | 1608 (C=0) | 3.27 ($CH_3$, 3H, s) |

- continued -

- continued -

| Derivative No. | $R^1$ | $R^2$ | A | Crystal form | Thin layer chromatography Rf | Thin layer chromatography Solvent* system | Infrared absorption spectrum ($cm^{-1}$) | Chemical shift of nuclear magnetic resonance spectrum derived from the amine induced (δ, ppm**) |
|---|---|---|---|---|---|---|---|---|
| 15 | $COCH_3$ | H | 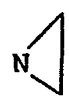 | amorphous | 0.33 | B | 1606 (C=0) | 3.30 ($CH_2CH_2$, 4H, br) |
| 16 | $COCH_3$ | H | 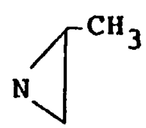 | amorphous | 0.15 | A | 1602 (C=0) | 1.43 ($CH_3$, 3H, d) and 3.30 ($CH_2$, 2H, br) |
| 17 | $COCH_3$ | H | 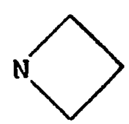 | flake | 0.30 | B | 1610 (C=0) | 4.20 ($CH_2N—CH_2$, 4H, t) |
| 18 | $COCH_3$ | H | 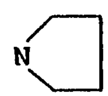 | flake | 0.29 | B | 1600 (C=0) | 3.55 ($CH_2NCH_2$, 4H, br) |

- continued -

- continued -

| Derivative No. | $R^1$ | $R^2$ | A | Crystal form | Thin layer chromatography: Rf | Thin layer chromatography: Solvent system* | Infrared absorption spectrum ($cm^{-1}$) | Chemical shift of nuclear magnetic resonance spectrum derived from the amine induced (δ, ppm**) |
|---|---|---|---|---|---|---|---|---|
| 19 | $COCH_3$ | H | 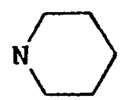 | flake | 0.34 | B | 1600 (C=0) | 3.53 ($CH_2NCH_2$, 4H, br) |
| 20 | $COCH_3$ | H | 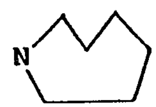 | amorphous | 0.45 | C | 1600 (C=0) | 3.53 ($CH_2NCH_2$, 4H, br) |
| 21 | $COCH_3$ | H | 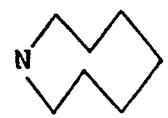 | amorphous | 0.39 | B | 1604 (C=0) | 3.67 ($CH_2NCH_2$, 4H, br) |
| 22 | $COCH_3$ | H | 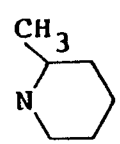 | flake | 0.16 | A | 1600 (C=0) | 1.27 ($CH_3$, 3H, d) |

- continued -

- continued -

| Derivative No. | $R^1$ | $R^2$ | A | Crystal form | Thin layer chromatography Rf | Thin layer chromatography Solvent system* | Infrared absorption spectrum ($cm^{-1}$) | Chemical shift of nuclear magnetic resonance spectrum derived from the amine induced (δ, ppm**) |
|---|---|---|---|---|---|---|---|---|
| 23 | $COCH_3$ | H | 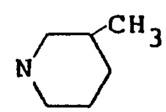 | flake | 0.35 | B | 1602 (C=0) | 1.00 ($CH_3$, 3H, d) |
| 24 | $COCH_3$ | H | 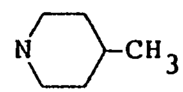 | flake | 0.35 | B | 1600 (C=0) | 1.03 ($CH_3$, 3H, d) |
| 25 | $COCH_3$ | H | 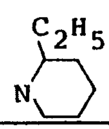 | flake | 0.22 | A | 1600 (C=0) | 0.95 ($CH_3$, 3H, m) |
| 26 | $COCH_3$ | H | 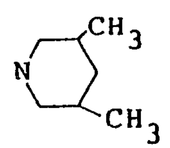 | needle | 0.24 | A | 1600 (C=0) | 1.03 ($CH_3$, 6H, d) |

- continued -

- continued -

| Derivative No. | $R^1$ | $R^2$ | A | Crystal form | Thin layer chromatography Rf | Solvent system* | Infrared absorption spectrum ($cm^{-1}$) | Chemical shift of nuclear magnetic resonance spectrum derived from the amine induced (δ, ppm**) |
|---|---|---|---|---|---|---|---|---|
| 27 | $COCH_3$ | H | 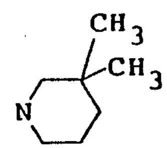 | flake | 0.25 | A | 1600 (C=0) | 1.00 ($CH_3$, 6H, s) |
| 28 | $COCH_3$ | H | 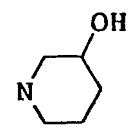 | flake | 0.19 | C | 1599 (C=0) | 3.30 ($CH_2NCH_2$, 4H, br) |
| 29 | $COCH_3$ | H | 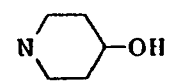 | flake | 0.12 | C | 1599 (C=0) | 3.30 ($CH_2NCH_2$, 4H, br) |
| 30 | $COCH_3$ | H | 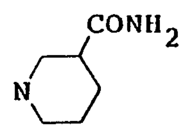 | flake | 0.35 | C | 1600 (C=0) | 3.20 ($CH_2NCH_2$, 4H, br) |

- continued -

- continued -

| Derivative No. | $R^1$ | $R^2$ | A | Crystal form | Thin layer chromatography Rf | Solvent system* | Infrared absorption spectrum ($cm^{-1}$) | Chemical shift of nuclear magnetic resonance spectrum derived from the amine induced (δ, ppm**) |
|---|---|---|---|---|---|---|---|---|
| 31 | $COCH_3$ | H | 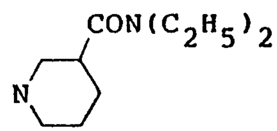 | flake | 0.27 | B | 1600 (C=0) | 1.20 ($CH_3$, 6H, q) |
| 32 | $COCH_3$ | H | 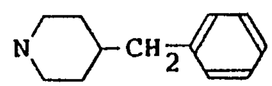 | flake | 0.41 | B | 1600 (C=0) | 7.23 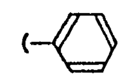 , 5H, m) |
| 33 | $COCH_3$ | H | 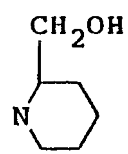 | flake | 0.11 | B | 1599 (C=0) | |
| 34 | $COCH_3$ | H | 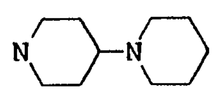 | flake | 0.33 | D | 1599 (C=0) | 1.53 ($CH_2CH_2CH_2$ 6H, br) |

- continued -

- continued -

| Derivative No. | $R^1$ | $R^2$ | A | Crystal form | Thin layer chromatography: Rf | Thin layer chromatography: Solvent system* | Infrared absorption spectrum ($cm^{-1}$) | Chemical shift of nuclear magnetic resonance spectrum derived from the amine induced (δ, ppm**) |
|---|---|---|---|---|---|---|---|---|
| 35 | $COCH_3$ | H | 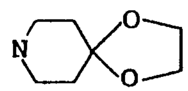 | flake | 0.31 | B | 1599 (C=O) | 4.00 ($OCH_2CH_2O$, 4H, s) |
| 36 | $COCH_3$ | H | 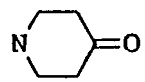 | flake | 0.21 | B | 1601 (C=O) | 2.67 ($CH_2\overset{\overset{O}{\|}}{C}CH_2$, 4H, t) and 3.95 ($CH_2NCH_2$, 4H, br) |
| 37 | $COCH_3$ | H | 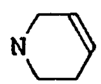 | flake | 0.43 | C | 1600 (C=O) | 3.70 and 4.00 ($CH_2NCH_2$, 4H, br) |
| 38 | $COCH_3$ | H | 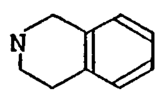 | flake | 0.43 | B | 1600 (C=O) | 3.75 ($NCH_2CH_2$, 2H, br) and 7.16 (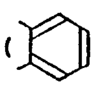, 4H, s) |

- continued -

- continued -

| Derivative No. | $R^1$ | $R^2$ | A | Crystal form | Thin layer chromatography Rf | Solvent* system | Infrared absorption spectrum ($cm^{-1}$) | Chemical shift of nuclear magnetic resonance spectrum derived from the amine induced (δ, ppm**) |
|---|---|---|---|---|---|---|---|---|
| 39 | $COCH_3$ | H | 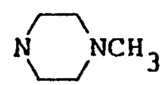 | flake | 0.32 | D | 1600 (C=0) | 2.63 ($CH_2N(CH_3)CH_2$, 4H, br) and 3.63 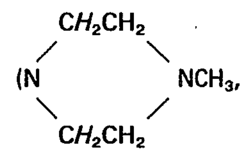 4H, br) |
| 40 | $COCH_3$ | H | 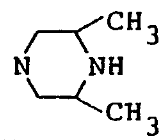 | flake | 0.34 | D | 1600 (C=0) | 1.20 ($CH_3$, 6H, d) and 3.80 ($CH_2NCH_2$, 4H, d) |
| 41 | $COCH_3$ | H | 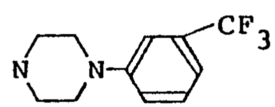 | amorphous | 0.44 | B | 1598 (C=0) | 3.53 ($CH_2NCH_2$, 4H, br) and 7.10 (aromatic ring, 4H, br) |

- continued -

- continued -

| Derivative No. | $R^1$ | $R^2$ | A | Crystal form | Thin layer chromatography Rf | Solvent system* | Infrared absorption spectrum ($cm^{-1}$) | Chemical shift of nuclear magnetic resonance spectrum derived from the amine induced (δ, ppm**) |
|---|---|---|---|---|---|---|---|---|
| 42 | $COCH_3$ | H | 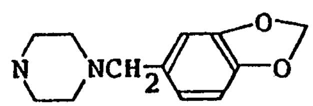 | flake | 0.28 | B | 1602 (C=0) | 5.93 ($CH_2$– 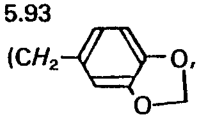 2H, s), 6.72 (aromatic ring, 2H, s) and 6.83 (aromatic ring, 1H, s) |
| 43 | $COCH_3$ | H | 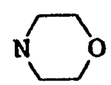 | flake | 0.22 | B | 1599 (C=0) | 3.56 ($CH_2NCH_2$, 4H, br) and 3.90 ($CH_2OCH_2$, 4H, br) |
| 44 | $COCH_3$ | H | 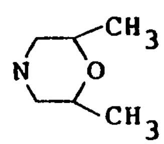 | flake | 0.29 | B | 1599 (C=0) | 1.30 ($CH_3$, 6H, d) and 3.73 ($CH_2NCH_2$, 4H, br) |

- continued -

- continued -

| Derivative No. | $R^1$ | $R^2$ | A | Crystal form | Thin layer chromatography Rf | Solvent* system | Infrared absorption spectrum ($cm^{-1}$) | Chemical shift of nuclear magnetic resonance spectrum derived from the amine induced (δ, ppm**) |
|---|---|---|---|---|---|---|---|---|
| 45 | $COCH_3$ | H | 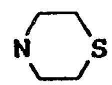 | flake | 0.34 | B | 1601 (C O) | 2.73 ($CH_2SCH_2$, 4H, br) and 3.90 ($CH_2NCH_2$, 4H, br) |
| 46 | $COCH_3$ | OH | 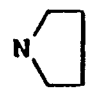 | flake | 0.28 | B | 1610 (C O) | 3.20 ($CH_2NCH_2$, 4H, b) |
| 47 | $COCH_3$ | OH | 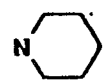 | flake | 0.43 | B | 1608 (C O) | 1.10 ($CH_2CH_2CH_2$, 6H, br) and 3.50 ($CH_2NCH_2$, 4H, br) |
| 48 | $COCH_3$ | OH | 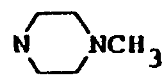 | flake | 0.26 | D | 1610 (C O) | 3.63 ($CH_2NCH_2$, 4H, br) |
| 49 | H | H | 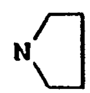 | flake | 0.58 | C | 1601 (C O) | 3.56 ($CH_2NCH_2$, 4H, br) |

- continued -

- continued -

| Derivative No. | $R^1$ | $R^2$ | A | Crystal form | Thin layer chromatography Rf | Solvent system* | Infrared absorption spectrum ($cm^{-1}$) | Chemical shift of nuclear magnetic resonance spectrum derived from the amine induced (δ, ppm**) |
|---|---|---|---|---|---|---|---|---|
| 50 | H | H | 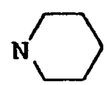 | flake | 0.66 | C | 1596 (C=0) | 3.63 ($CH_2NCH_2$, 4H, br) |
| 51 | H | H | 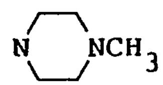 | flake | 0.23 | D | 1601 (C=0) | 3.63 ($CH_2NCH_2$, 4H, br) |

* A: chloroform/acetone = 9/1
B: chloroform/acetone = 8/2
C: chloroform/acetone = 7/3
D: chloroform/methanol = 9/1

** s: singlet
d: doublet
t: triplet
q: quartet
m: multiplet
br: broad

The rifamycin derivative of the present invention having the general formula (I) shows a strong antibacterial activity against the Gram-positive bacteria and the acid-fast bacteria. The antibacterial activity of the rifamycin derivative of the present invention having the general formula (I) is tested by the method according to the standard method of Japan Society of Chemotherapy [Chemotherapy (Tokyo), *29,* P76, (1981)]. The results obtained from the typical compounds are shown in Table 2 as the minimum inhibitory concentration (MIC, µg/ml). As shown in Table 2, it is clear that the rifamycin derivative of the present invention shows a strong antibacterial activity against the Gram-positive bacteria and the acid-fast bacteria. In Table 2, Test compound No. corresponds to derivative No. in Table 1.

It was also found that the rifamycin derivative of the present invention having the general formula (I) had a low toxicity since toxicity was never exhibited by oral administration of 1000 mg/kg weight of the rifamycin derivative of the present invention having the general formula (I) to mice.

TABLE 2

| | Test compound | | | | | | |
|---|---|---|---|---|---|---|---|
| Test organism | 2 | 3 | 4 | 9 | 11 | 12 | 17 |
| *Micrococcus luteus* IFO 12708 | 0.02≧ | 0.02≧ | 0.02≧ | 0.02≧ | 0.02≧ | 0.02≧ | 0.02≧ |
| *Bacillus subtilis* IFO 3134 | 0.04 | 0.02≧ | 0.08 | 0.02≧ | 0.02≧ | 0.02≧ | 0.02≧ |
| *Staphylococcus aureus* IFO 12732 | 0.02≧ | 0.02≧ | 0.02≧ | 0.02≧ | 0.02≧ | 0.02≧ | 0.02≧ |
| *Escherichia coli* IFO 12734 | 1.25 | 2.5 | >10 | 5 | >10 | >10 | 2.5 |
| *Klebsiella pneumoniae* IFO 3512 | 2.5 | 5 | >10 | 10 | 5 | >10 | 2.5 |
| *Mycobacterium smegmatis* ATCC 607 | 1.25 | 1.25 | 1.25 | 1.25 | 2.5 | 1.25 | 0.63 |

TABLE 2 (continued)

| | Test compound | | | | | | |
|---|---|---|---|---|---|---|---|
| Test organism | 18 | 19 | 20 | 23 | 24 | 27 | 31 |
| *Micrococcus luteus* IFO 12708 | 0.02≧ | 0.02≧ | 0.02≧ | 0.02≧ | 0.02≧ | 0.02≧ | 0.02≧ |
| *Bacillus subtilis* IFO 3134 | 0.02≧ | 0.02≧ | 0.04 | 0.02≧ | 0.02≧ | 0.02≧ | 0.02≧ |
| *Staphylococcus aureus* IFO 12732 | 0.02≧ | 0.02≧ | 0.08 | 0.02≧ | 0.02≧ | 0.02≧ | 0.02≧ |
| *Escherichia coli* IFO 12734 | 2.5 | 2.5 | 1.25 | >10 | >10 | >10 | 5 |
| *Klebsiella pneumoniae* IFO 3512 | 5 | 10 | >10 | >10 | >10 | >10 | >10 |
| *Mycobacterium smegmatis* ATCC 607 | 1.25 | 1.25 | 1.25 | 1.25 | 1.25 | 1.25 | 1.25 |

TABLE 2 (continued)

| Test organism | Test compound 37 | 39 | 45 | 46 | 49 | Rifampicin (control) |
|---|---|---|---|---|---|---|
| *Micrococcus luteus* IFO 12708 | 0.02≧ | 0.02≧ | 0.02≧ | 0.02≧ | 0.02≧ | 0.02≧ |
| *Bacillus subtilis* IFO 3134 | 0.02≧ | 0.04 | 0.04 | 0.02≧ | 0.02≧ | 0.04 |
| *Staphylococcus aureus* IFO 12732 | 0.08 | 0.04 | 0.02≧ | 0.02≧ | 0.02≧ | 0.02≧ |
| *Escherichia coli* IFO 12734 | 2.5 | 0.63 | 2.5 | >10 | 1.25 | 10 |
| *Klebsiella pneumoniae* IFO 3512 | >10 | 1.25 | 10 | >10 | 5 | 5 |
| *Mycobacterium smegmatis* ATCC 607 | 1.25 | 1.25 | 1.25 | 1.25 | 1.25 | 10 |

The rifamycin derivative having the general formula (II), which is a starting material for preparing the rifamycin derivative of the present invention having the general formula (I), can scarcely be absorbed after oral administration, while the rifamycin derivative of the present invention having the general formula (I) is absorbed to show a good high blood level. Typical compounds were tested employing Wistar Strain male rats weighing 270 to 300 g and the results obtained are shown in Table 3. The test was carried out according to the unsual method, by means of bioassay employing *Micrococcus luteus* IFO 12708 as an assay bacteria.

TABLE 3

| Derivative No. | Dose (mg/kg) | Concentration in serum (µg/m) 1 hr. | 3 hrs. | 5 hrs. |
|---|---|---|---|---|
| 2 | 20 | 3.0 | 5.4 | 3.0 |
| 3 | 20 | 1.8 | 3.3 | 1.3 |
| 4 | 20 | 2.0 | 8.5 | 2.1 |
| 9 | 20 | 2.1 | 5.4 | 1.8 |
| 11 | 20 | 2.9 | 3.4 | 1.0 |
| 17 | 20 | 4.1 | 3.7 | 2.5 |
| 18 | 20 | 2.3 | 4.9 | 3.4 |
| 19 | 20 | 4.9 | 12.4 | 16.6 |
| 20 | 20 | 3.2 | 5.7 | 4.9 |
| 23 | 20 | 2.1 | 6.1 | 5.2 |
| 25 | 20 | 2.4 | 6.6 | 3.5 |
| 27 | 20 | 1.8 | 6.4 | 1.7 |
| 49 | 20 | 2.7 | 3.5 | 2.8 |
| 50 | 20 | 0.7 | 2.4 | 2.8 |
| Benzoxazino-rifamycin (GB—A—1 081 757) | 100 | n.d. | n.d. | n.d. |

n.d.: not detected

Antibacterial agents containing the rifamycin derivative of the present invention as an effective component can be in any dosage form of an oral, rectal, topical or parenteral administration. Examples of the dosage form are, for instance, tablets, capsules, granules, syrups, suppositories, ointments and the like. Carrier used in the dosage form of the antibacterial agents of the present invention is usually inactive pharmaceutical carrier of an organic or an inorganic solid or liquid suitable for an oral, rectal, topical or parenteral administration such as, for instance, crystalline cellulose, gelatin, lactose, starch, magnesium stearate, talc, plant or animal fat or oil, gum or polyalkylene glycol. A ratio of the antibacterial agent of the present invention to the carrier in the dosage form can vary from 0.2 to 100% by weight. The antibacterial agent of the present invention can contain another pharmaceutical component such as another antibacterial agent compatible with the antibacterial agent of the present invention. In this case, however, the antibacterial agent of the present invention is not necessarily the chief ingredient of the dosage form.

The antibacterial agent of the present invention is administered with such a dose that the desired activity is achieved without any side-effect. Though the actual dosage should be determined according to the judgment of the doctor, around 10 mg to around 10 g, preferably around 20 mg to around 5 g per day of the antibacterial agent of the present invention is usually administered for adults. The antibacterial agent of the present invention can be administered in a pharmaceutical dosage unit containing 1 mg to 5 g, preferably 3 mg to 1 g of an effective component.

The present invention is more particularly described and explained by the following Examples. However, it should be understood that the present invention is not limited to such Examples and various changes and modifications can be made without departing from the scope and spirit of the present invention.

In the following Examples, infrared absorption spectrum was measured according to the potassium bromide tablet method. Thin layer chromatography was carried out using Silica gel 60 $F_{254}$ (E. Merck Co.) and the thin layer chromatography plate (20 cm × 20 cm). Nuclear magnetic resonance spectrum was measured using tetramethylsilane as an internal standard and deuterated chloroform solution. Measurement of the visible absorption spectrum was conducted in a solvent of methanol.

Example 1

[Synthesis of derivative No. 1]

A solution of 0.2 g of benzoxazinorifamycin, which was prepared according to the method described in *Helv. Chim. Acta, 56,* p 2369 (1973), in 7.0 ml of methanol was mixed with a solution of 0.2 g of dimethylamine hydrochloride and 0.28 ml of triethylamine. The mixture was stirred for 18 hours at room temperature. Ethyl acetate was added to the reaction mixture and the resultant was washed twice with a saturated NaCl solution, followed by distillation of a solvent under reduced pressure. The residue was subjected to silica-gel column-chromatography [adsorbent: Wakogel® C—200, eluent: chloroform-acetone (90:10)] to give a fraction containing the desired derivative. A solvent of the fraction was distilled away under reduced pressure and the residue was subjected to silica-gel column-chromatography as above, followed by work-up of the resultant to give 53 mg of a pure derivative No. 1.

Infrared absorption spectrum (ν $cm^{-1}$)): 3450, 2960, 2930, 1720, 1660, 1560, 1490, 1460, 1410, 1360, 1310, 1260, 1200, 1170, 1120, 1060, 1040, 980, 950, 910, 820, 770 and 700

Rf = 0.26, blue spot [chloroform-acetone (80:20)]

Nuclear magnetic resonance spectrum δ (ppm) ($CDCl_3$): 0.55, 0.80, 0.95 ($CHCH_3$), 2.05, 2.10, 2.15, 2.20, 3.05, 3.16 ($CH_3$), around 4.80 to 5.20 (protons at 25-position and 28-position), around 5.90 to 6.20, 6.55, 6.75, 6.85, 7.80 (protons at 17-position, 19-position and 29-position and protons of benzoxazine ring), 7.75 (amide proton) and 15.1 (phenolic proton)

Visible absorption spectrum [λ max. nm ($E^{1\%}_{1cm}$)]: 361 (154), 481 (51) and 633 (436)

Elementary analysis for $C_{45}H_{53}N_3O_{12}$

Calcd. (%): C 65.28, H 6.45, N 5.08
Found (%): C 65.35, H 6.51, N 5.01

Example 2

[Synthesis of derivative No. 2]

A solution of 1.0 g of benzoxazinorifamycin in 5 ml of dimethylsulfoxide (hereinafter referred to as "DMSO") was mixed with 0.22 ml of ethylmethylamine and 1.0 g of manganese dioxide. The mixture was stirred at room temperature for 5 hours. After completion of the reaction, the reaction mixture was diluted by addition of 50 ml of ethyl acetate and then manganese dioxide was filtered. A filtrate was washed successively with water (twice), diluted hydrochloric acid (twice) and water (twice) and a solvent was distilled away under reduced pressure. The residue was subjected to silica-gel column-chromatography (filler: Wakogel® C—200, developer: ethyl acetate) to give a fraction containing the desired derivative. A solvent of the fraction was distilled away under reduced pressure and the obtained residue was dissolved in ethyl acetate, to which n-hexane was added to crystallize 0.73 g of the desired derivative No. 2.

Infrared absorption spectrum (ν $cm^{-1}$): 3460, 2960, 2930, 1715, 1655, 1600, 1560, 1490, 1460, 1415, 1390, 1375, 1350, 1305, 1255, 1170, 1125, 1080, 1060, 1035, 970, 940, 910, 895, 815, 762, 695 and 440

Nuclear magnetic resonance spectrum δ (ppm) ($CDCl_3$): 0.55, 0.78, 0.93 ($CHCH_3$), 1.27 ($—NCH_2CH_3$)], 1.80, 2.01, 2.11, 2.14, 3.04 ($CH_3$), 3.13 ($N—CH_3$), 3.56 ($—NCH_2CH_3$), round 4.85 to 5.20 (protons at 25-position

and 28-position), around 5.85 to 6.15, 6.53, 6.76, 6.86, 7.78 (protons at 17-position, 19-position and 29-position and protons of benzoxazine ring), 7.68 (amide proton) and 15.07 (phenolic proton)

Visible absorption spectrum [λ max. nm ($E^{1\%}_{1cm}$)]: 363 (176), 480 (53) and 637 (504)

Elementary analysis for $C_{46}H_{55}N_3O_{12}$

Calcd. (%): C 65.62, H 6.59, N 4.99

Found (%): C 65.42, H 6.71, N 5.15

Example 3

[Synthesis of derivative No. 3]

A solution of 2.0 g of benzoxazinorifamycin in 10 ml of DMSO was mixed with 0.53 ml of diethylamine and 2.0 g of manganese dioxide. The mixture was stirred at room temperature for two days. After completion of the reaction, the procedure of Example 2 was repeated to give 0.79 g of the desired derivative No. 3.

Infrared absorption spectrum (ν $cm^{-1}$): 3450, 2970, 2940, 1715, 1663, 1650, 1600, 1560, 1520, 1490, 1470, 1403, 1380, 1355, 1308, 1260, 1185, 1175, 1122, 1078, 1040, 975, 945, 920, 900, 820, 765, 695 and 445

Nuclear magnetic resonance spectrum δ (ppm) ($CDCl_3$): 0.55, 0.76, 0.91 ($CHCH_3$), 1.28 ($N—CH_2—CH_3$), 1.79, 2.00, 2.10, 2.14, 3.03 ($CH_2$), 3.54 ($N—CH_2—CH_3$), around 4.80 to 5.15 (protons at 25-position and 28-position), around 5.85 to 6.15, 6.48, 6.75, 6.85, 7.71, 7.81 (protons at 17-position, 19-position and 29-position and protons of benzoxazine ring), 7.71 (amide proton) and 15.13 (phenolic proton)

Visible absorption spectrum [λ max. nm ($E^{1\%}_{1cm}$)]: 363 (183), 480 (49) and 642 (546)

Elementary analysis for $C_{47}H_{57}N_3O_{12}$

Calcd. (%): C 65.95, H 6.71, N 4.91

Found (%): C 65.74, H 6.78, N 4.76

Example 4

[Synthesis of derivative No. 4]

A solution of 2.0 g of benzoxazinorifamycin in 10 ml of DMSO was mixed with a 0.70 ml of dipropylamine and 2.0 g of manganese dioxide. The mixture was stirred at room temperature for two days. After completion of the reaction, the procedure of Example 2 was repeated to give 0.72 g of the desired derivative No. 4.

Infrared absorption spectrum (ν $cm^{-1}$): 3460, 2960, 2930, 2870, 1718, 1645, 1601, 1562, 1520, 1488, 1465, 1405, 1365, 1310, 1280, 1240, 1170, 1125, 1100, 1060, 1035, 975, 945, 925, 910, 845, 820, 770 and 450

Nuclear magnetic resonance spectrum δ (ppm) ($CDCl_3$): 0.56, 0.75 0.92 ($CHCH_2$), 1.00 ($NCH_2CH_2CH_3$), around 1.45 to 2.25 ($N—CH_2CH_2CH_3$), 1.79, 2.00, 2.09, 2.17, 3.03 ($CH_3$), 3.37 ($N—CH_2CH_2CH_3$), around 4.75 to 5.15 (protons at 25-position and 28-position), around 5.75 to 6.15, 6.44, 6.72, 6.83, 7.35, 7.44 (protons at 17-position, 19-position and 29-position and protons of benzoxazine ring), 7.30 (amide proton) and 15.06 (phenolic proton)

Visible absorption spectrum [λ max. nm ($E^{1\%}_{1cm}$)]: 364 (170), 482 (49) and 644 (519)

Elementary analysis for $C_{49}H_{61}N_3O_{12}$

Calcd. (%): C, 66.57, H 6.96, N 4.75

Found (%): C 66.73, H 6.79, N 4.93

Example 5

[Synthesis of derivative No. 5]

A solution of 1.0 g of benzoxazinorifamycin in 5 ml of DMSO was mixed with 0.22 ml of N-methylpropargylamine and 1.0 g of manganese dioxide. The mixture was stirred at room temperature for 6 hours. After completion of the reaction, the procedure of Example 2 was repeated to give 0.09 g of the desired derivative No. 5.

Example 6

[Synthesis of derivative No. 6]

A solution of 1.0 g of benzoxazinorifamycin in 5 ml of DMSO was mixed with 0.24 ml of 3-methylaminopropionitrile and 1.0 g of manganese dioxide. The mixture was stirred at room temperature for 3 days. After completion of the reaction, the procedure of Example 2 was repeated to give 0.61 g of the desired derivative No. 6.

Example 7

[Synthesis of derivative No. 7]

A solution of 1.0 g of benzoxazinorifamycin in 10 ml of methanol was mixed with 1.65 ml of N,N,N'-trimethylethylenediamine and 2.2 ml of a solution of 1.2 N hydrochloric acid in methanol. The mixture was stirred at room temperature for 17 hours. To the reaction mixture was added 0.5 g of manganese dioxide and the mixture was stirred at room temperature for 5 minutes. The resultant was filtered and chloroform was added to the filtrate, which was then washed twice with a saturated NaCl solution. A solvent was distilled away under reduced pressure and the residue was purified by repeating the procedure of silica-gel column-chromatography [eluent: chloroform-methanol (90:10)] to give 0.32 g of a pure derivative No. 7.

Infrared absorption spectrum (ν $cm^{-1}$): 3450, 2980, 2950, 1720, 1660, 1600, 1560, 1500, 1460, 1420, 1390, 1370, 1310, 1250, 1200, 1170, 1130, 1110, 1060, 1040, 980, 950, 920, 820, 780 and 700

Rf = 0.34, blue spot [chloroform-methanol (90:10)]

Nuclear magnetic resonance spectrum δ (ppm) ($CDCl_3$): 0.50, 0.75, 0.90 ($CHCH_3$), 1.83, 1.86, 2.01, 2.15, 2.20, 2.33, 2.60, 3.02 ($CH_3$ or protons of ethylenediamino group), around 4.85 to 5.20 (protons at 25-position and 28-position), around 5.90 to 6.90 (protons at 17-position, 19-position and 29-position and protons of benzoxazine ring), 7.75 (proton of benzoxazine ring), 8.06 (amide proton) and 15.2 (phenolic proton)

Visible absorption spectrum [λ max. nm ($E^{1\%}_{1cm}$)]: 361 (150),481 (56) and 630 (437)

Elementary analysis for $C_{48}H_{60}N_4O_{12}$

Calcd. (%): C 65.14, H 6.83, N 6.33
Found (%): C 65.07, H 6.71, N 6.42

Example 8

[Synthesis of derivative No. 8]

A solution of 1.0 g of benzoxazinorifamycin in 5 ml of DMSO was mixed with 0.21 ml of 2-(methylamino)-ethanol and the mixture was stirred at room temperature for 8 hours. After completion of the reaction, the procedure of Example 2 was repeated to give 0.62 g of the desired derivative compound No. 8.

Example 9

[Synthesis of derivative No. 9]

A solution of 1.0 g of benzoxazinorifamycin in 5 m of DMSO was mixed with 0.41 g of bis(ethoxyethyl)-amine and the mixture was stirred at room temperature for two days. After completion of the reaction, the procedure of Example 2 was repeated to give 0.13 g of the desired derivative No. 9.

Infrared absorption spectrum (ν $cm^{-1}$): 3470, 2980, 2940, 2880, 1718, 1662, 1601, 1560, 1520, 1485, 1460, 1400, 1380, 1355, 1320, 1260, 1230, 1170, 1120, 1060, 1040, 980, 950, 915, 900, 810, 765, 695 and 445

Nuclear magnetic resonance spectrum δ (ppm ($CDCl_3$): 0.57, 0.75, 0.92 ($CHCH_3$), 1.20 ($-OCH_2CH_3$), 1.78, 2.00, 2.10, 2.16, 3.05 ($CH_3$), around 3.30 to 3.85 ($-NCH_2CH_2OCH_2CH_3$), around 4.80 to 5.15 (protons at 25-position and 28-position), around 5.85 to 6.15, 6.57, 6.85, 6.96, 7.71, 7.81 (protons at 17-position, 19-position and 29-position and protons of benzoxazine ring), 7.55 (amide proton) and 15.00 (phenolic proton)

Visible absorption spectrum [λ max. nm ($E^{1\%}_{1cm}$)]: 362 (156), 482 (54) and 635 (460)

Elementary analysis for $C_{51}H_{65}N_3O_{14}$

Calcd. (%): C 64.88, H 6.94, N 4.45
Found (%): C 64.70, H 6.84, N 4.68

Example 10

[Synthesis of derivative No. 10]

A solution of 1.0 g of benzoxazinorifamycin in 5 ml of DMSO was mixed with 0.64 ml of N-(3-methoxypropyl)-3,4,5-trimethoxybenzylamine and 1.0 g of manganese dioxide and the mixture was stirred at room temperature for two days. After completion of the reaction, the procedure of Example 2 was repeated to give 0.11 g of the desired derivative No. 10.

Example 11

[Synthesis of derivative No. 11]

A solution of 1.0 g of benzoxazinorifamycin in 5 m of DMSO was mixed with 0.33 ml of methylamino-acetaldehyde dimethyl acetal and 1.0 g of manganese dioxide and the mixture was stirred at room temperature for 24 hours. After completion of the reaction, the procedure of Example 2 was repeated to give 0.55 g of the desired derivative No. 11.

Infrared absorption spectrum (ν $cm^{-1}$): 3450, 2970, 2930, 2830, 1715,. 1645, 1600, 1560, 1520, 1490, 1465, 1410, 1380, 1370, 1305, 1255, 1200, 1170, 1130, 1110, 1060, 1035, 975, 910, 855, 820, 765, 710 and 445

Nuclear magnetic resonance spectrum δ (ppm) ($CDCl_3$): 0.53, 0.77, 0.93 ($CHCH_3$), 1.82, 2.01, 2.13, 2.14, 3.06 ($CH_3$), 3.20 ($N-CH_3$), 3.43 ($OCH_3$), 3.60 ($-CH-(OCH_3)_2$), 4.56 ($-NCH_2-$), around 4.85 to 5.20 (protons at 25-position and 28-position), around 5.95 to 6.10, 6.60, 6.85, 6.96, 7.75, 7.85 (protons at 17-position, 19-position and 27-position and protons of benzoxazine ring), 7.58 (amide proton) and 14.9 (phenolic proton)

Visible absorption spectrum [λ max. nm ($E^{1\%}_{1cm}$)] 361 (154), 480 (58) and 630 (431)

Elementary analysis for $C_{48}H_{59}N_3O_{14}$

Calcd. (%): C 63.92, H 6.59, N 4.66
Found (%): C 64.10, H 6.73, N 4.54

Example 12

[Synthesis of derivative No. 12]

A solution of 1.0 g of benzoxazinorifamycin in 5 ml of DMSO was mixed with 0.33 ml of N-methylcyclohexylamine and 1.0 g of manganese dioxide and the mixture was stirred at room temperature for two days. After completion of the reaction, the procedure of Example 2 was repeated to give 0.27 g of the desired derivative No. 12.

Example 13

[Synthesis of derivative No. 13]

A solution of 1.0 g benzoxazinorifamycin in 5 ml of DMSO was mixed with 0.37 ml of 1-methyl-4-(methylamino)piperidine and 1.0 g of manganese dioxide and the mixture was stirred at room temperature for two days. After completion of the reaction, the procedure of Example 2 was repeated to give 0.17 g of the desired derivative No. 13.

Example 14

[Synthesis of derivative No. 14]

A solution of 1.0 g of benzoxazinorifamycin in 5 ml of DMSO was mixed with 0.5 g of N-methyl-D-glucamine and 1.0 g of manganese dioxide and the mixture was stirred at room temperature for 4 hours. After completion of the reaction, the procedure of Example 2 was repeated to give 0.63 g of the desired derivative No. 14.

Example 15

[Synthesis of derivative No. 15]

A solution of 1.0 g of benzoxazinorifamycin in 5 ml of DMSO was mixed with 0.13 ml of ethylenimine and 1.0 g of manganese dioxide and the mixture was stirred at room temperature for one day. After completion of the reaction, the procedure of Example 2 was repeated to give 0.28 g of the desired derivative No. 15.

Example 16

[Synthesis of derivative No. 16]

A solution of 1.0 g of benzoxazinorifamycin in 5 ml of DMSO was mixed with 0.18 ml of propyleneimine and 1.0 g of manganese dioxide and the mixture was stirred overnight at room temperature. After completion of the reaction, the procedure of Example 2 was repeated to give 0.1 g of the desired derivative No. 16.

Example 17

[Synthesis of derivative No. 17]

A solution of 1.0 g of benzoxazinorifamycin in 5 ml of DMSO was mixed with 0.17 ml of trimethylene-imine and 1.0 g of manganese dioxide and the mixture was stirred at room temperature for 6 hours. After completion of the reaction, the procedure of Example 2 was repeated to give 0.35 g of the desired derivative No. 17.

Infrared absorption spectrum [$\dot{\nu}$ $cm^{-1}$]: 3450, 2970, 2940, 2870, 1720, 1650, 1610, 1565, 1498, 1470, 1400, 1380, 1312, 1260, 1175, 1135, 1115, 1065, 1040, 975, 950, 920, 825, 818, 765, 670 and 435

Nuclear magnetic resonance spectrum δ (ppm) ($CDCl_3$): 0.52, 0.77, 0.94 ($CHCH_3$), 1.25 (protons of azetidine ring), 1.80, 2.02, 2.08, 2.84, 3.05 ($CH_3$), 4.20, ($-CH_2NCH_2-$), around 4.75 to 5.15 (protons at 25-position and 28-position), around 5.85 to 6.15, 6.20, 6.38, 6.48, 7.68, 7.77 (protons at 17-position, 19-position and 29-position and protons of benzoxazine ring), 7.72 (amide proton) and 15.07 (phenolic proton)

Visible absorption spectrum [λ max. nm ($E^{1\%}_{1cm}$)]: 362 (178), 476 (58) and 640 (522)

Elementary analysis for $C_{46}H_{53}N_3O_{12}$

Calcd. (%): C 65.78, H 6.36, N 5.00

Found (%): C 65.89, H 6.50, N 4.86

Example 18

[Synthesis of derivative No. 18]

A solution of 0.8 g of benzoxazinorifamycin in 10 ml of DMSO was mixed with 0.83 ml of pyrrolidine and 1.7 ml of a solution of 1.2 N hydrochloric acid in methanol and the mixture was stirred at room temperature for 17 hours. After completion of the reaction, the procedure of Example 1 was repeated to give 0.07 g of the desired derivative No. 18.

Infrared absorption spectrum (ν $cm^{-1}$): 3450, 2970, 2920, 2850, 1720, 1660, 1600, 1550, 1480, 1460, 1390, 1370, 1340, 1320, 1260, 1220, 1160, 1130, 1060, 1040, 980, 940, 920, 910, 860, 820, 770 and 600

Rf = 0.38, blue spot [chloroform-acetone (70:30)]

Nuclear magnetic resonance spectrum δ (ppm) ($CDCl_3$): 0.52, 0.80, 0.90 ($CHCH_3$), 1.80, 2.00, 2.10, 2.18, 3.02, 3.55 ($CH_3$ and protons of pyrrolidine ring), around 4.80 to 5.10 (protons at 25-position and 28-position), 5.95, 6.06, 6.50, 6.66, 6.75, 7.70 (protons at 17-position, 19-position and 29-position and protons of benzoxazine ring), 8.25 (amide proton) and 15.2 (phenolic proton)

Visible absorption spectrum [λ max. nm ($E^{1\%}_{1cm}$)]: 363 (138), 480 (43) and 642 (409)

Elementary analysis for $C_{47}H_{55}N_3O_{12}$

Calcd. (%): C 66.11, H 6.49, N 4.92

Found (%): C 66.03, H 6.56, N 4.84

Example 19

[Synthesis of derivative No. 19]

A solution of 5.5 g of benzoxazinorifamycin in 70 ml of methanol was mixed with 6.0 g of piperidine and 11.7 ml of a solution of 1.2 N hydrochloric acid in methanol and the mixture was stirred at room temperature for 7 hours. After completion of the reaction, 2.8 g of manganese dioxide was added to the reaction mixture and the mixture was stirred at room temperature for 5 minutes. The resultant was filtered and chloroform was added to the filtrate, which was then washed twice with a saturated NaCl solution. A solvent was distilled away under reduced pressure and the residue was treated as in Example 2 to give 0.5 g of the desired derivative No. 19.

Infrared absorption spectrum (ν $cm^{-1}$): 3450, 2950, 2930, 2870, 1720, 1650, 1600, 1560, 1485, 1460, 1385, 1370, 1305, 1240, 1210, 1170, 1115, 1060, 1020, 980, 950, 920, 900, 850, 820, 770, 690, 640 and 580.

Rf = 0.45, blue spot [chloroform-aceton (70:30)]

Nuclear magnetic resonance spectrum δ (ppm) ($CDCl_3$): 0.53, 0.86, 0.93 ($CHCH_3$), 1.73, 1.80, 2.00, 2.10, 2.48, 3.03, 3.53 ($CH_3$ and protons of piperidine ring), around 4.85 to 5.06 (protons at 25-position and 28-position), 5.90, 6.03, 6.67, 6.69, 6.90, 7.00, 7.76 (protons at 17-position, 19-position and 29-position and protons of benzoxazine ring), 7.70 (amide proton) and 15.02 (phenolic proton)

Visible absorption spectrum [λ max. nm ($E_{1cm}^{1\%}$)]: 363 (160), 481 (55) and 643 (488)

Elementary analysis for $C_{48}H_{57}N_3O_{12}$

Calcd. (%): C 66.42, H 6.62, N 4.84

Found (%): C 66.30, H 6.73, N 4.95

Example 20

[Synthesis of derivative No. 20]

A solution of 5.5 g of benzoxazinorifamycin in 70 ml of methanol was mixed with 7.0 g of hexamethyleneimine and 11.7 ml of a solution of 1.2 N hydrochloric acid in methanol and the mixture was stirred at room temperature for 6 hours. After completion of the reaction, the procedure of Example 19 was repeated to give 0.4 g of the desired derivative No. 20.

Infrared absorption spectrum (ν $cm^{-1}$): 3460, 2960, 2930, 1720, 1660, 1650, 1600, 1560, 1520, 1490, 1460, 1400, 1360, 1305, 1250, 1205, 1160, 1125, 1100, 1060, 1040, 1000, 975, 945, 900, 860, 820, 770, 690, 640 and 580

Rf = 0.45, blue spot [chloroform-acetone (70:30)]

Nuclear magnetic resonance spectrum δ (ppm) ($CDCl_3$): 0.53, 0.75, 0.92 ($CHCH_3$), 1.65, 1.83, 2.02, 2.13, 3.06, 3.53 ($CH_3$ and protons of hexamethdyleneimine ring), around 4.85 to 5.10 (protons at 25-position and 28-position), 5.90, 6.05, 6.56, 6.59, 6.85, 6.92, 7.73, 7.85 (protons at 17-position, 19-position and 29-position and protons of benzoxazine ring), 7.67 (amide proton) and 15.13 (phenolic proton)

Visible absorption spectrum [λ max. nm ($E_{1cm}^{1\%}$)] 363 (178), 484 (55) and 644 (539)

Elementary analysis for $C_{49}H_{59}N_3O_{12}$

Calcd. (%): C 66.73, H 6.74, N 4.76

Found (%): C 66.86, H 6.67, N 4.84

Example 21

[Synthesis of derivative No. 21]

A solution of 5.5 g of benzoxazinorifamycin in 70 ml of methanol was mixed with 7.9 ml of heptamethyleneimino and 11.7 ml of a solution of 1.2 N hydrochloric acid in methanol and the mixture was stirred at room temperature for 6.5 hours. After completion of the reaction, the procedure of Example 19 was repeated to give 0.96 g of the desired derivative No. 21.

Example 22

[Synthesis of derivative No. 22]

A solution of 1.0 g of benzoxazinorifamycin in 5 ml of DMSO was mixed with 0.3 ml of 2-methylpiperazine and 1.0 g of manganese dioxide and the mixture was stirred at room temperature for two days. After completion of the reaction, the procedure of Example 2 was repeated to give 0.34 g of the desired derivative No. 22.

Example 23

[Synthesis of derivative No. 23]

A solution of 1.0 g of benzoxazinorifamycin in 5 ml of DMSO was mixed with 0.3 ml of 3-methylpiperidine and 1.0 g of manganese dioxide and the mixture was stirred at room temperature for 4 hours. After completion of the reaction, the procedure of Example 2 was repeated to give 0.46 g of the desired derivative No. 23.

Infrared absorption spectrum (ν $cm^{-1}$): 3450, 2970, 2940, 2880, 1720 1650, 1602, 1560, 1490, 1460, 1400, 1380, 1310, 1245, 1220, 1175, 1125, 1090, 1065, 1040, 970, 905, 860, 820, 770, 645 and 590

Nuclear magnetic resonance spectrum δ (ppm) ($CDCl_3$): 0.47, 0.77, 0.92 ($CHCH_3$), 1.00 ($CH_3$ of piperidine), around 1.45 to 2.25, around 2.55 to 3.25, around 3.75 to 4.10 (protons of piperidine ring), 1.80, 2.00, 2.10 2.14, 3.03 ($CH_3$), around 4.85 to 5.15 (protons at 25-position and 28-position), around 5.85 to 6.15,

6.67, 6.90, 7.01, 7.72, 7.82 (protons at 17-position, 19-position and 29-position and protons of benzoxazine ring), 7.82 (amide proton) and 15.01 (phenolic proton)

Visible absorption spectrum [λ max. nm ($E^{1\%}_{1\,cm}$)] 363 (163), 481 (52) and 646 (498)

Elementary analysis for $C_{49}H_{59}N_3O_{12}$

Calcd. (%): C 66.73, H 6.74, N 4.76
Found (%): C 66.61, H 6.96, N 4.87

Example 24

[Synthesis of derivative No. 24]

A solution of 1.0 g of benzoxazinorifamycin in 5 ml of DMSO was mixed with 0.3 ml of 4-methylpiperidine and 1.0 g of manganese dioxide and the mixture was stirred at room temperature for 4 hours. After completion of the reaction, the procedure of Example 2 was repeated to give 0.74 g of the desired derivative No. 24.

Example 25

[Synthesis of derivative No. 25]

A soltuon of 1.0 g of benzoxazinorifamycin in 5 ml of DMSO was mixed with 0.34 ml of 2-ethylpiperidine and 1.0 g of manganese dioxide and the mixture was stirred at room temperature for 5 days. After completion of the reaction, the procedure of Example 2 was repeated to give 0.22 g of the desired derivative No. 25.

Example 26

[Synthesis of derivative No. 26]

A solution of 1.0 g of benzoxazinorifamycin in 5 ml of DMSO was mixed with 0.34 ml of 3,5-dimethylpiperdine and 1.0 g of manganese dioxide and the mixture was stirred at room temperature for 4 hours. After completion of the reaction, the procedure of Example 2 was repeated to give 0.77 g of the desired derivative No. 26.

Example 27

[Synthesisis of derivative No. 27]

A solution of 1.0 g of benzoxazinorifamycin in 5 ml of DMSO was mixed with 0.32 ml of 3,3-dimethylpiperidine and 1.0 g of manganese dioxide and the mixture was stirred at room temperature for 2 hours. After completion of the reaction, the procedure of Example 2 was repeated to give 0.73 g of the desired derivative No. 27.

Infrared absorption spectrum (ν $cm^{-1}$): 3460, 2940, 2870, 1720, 1660, 1600, 1560, 1485, 1460, 1398, 1375, 1305, 1245, 1205, 1160, 1130, 1080, 1055, 1025, 980, 945, 898, 860, 815, 765, 710, 640 and 590

Nuclear magnetic resonance spectrum δ (ppm) ($CDCl_3$): 0.52, 0.77, 0.93 ($CHC\mathit{H}_3$), 1.00 ($CH—C\mathit{H}_3)_2$), around 1.40 to 1.80 (protons of piperidine ring), 1.79, 2.00, 2.10, 2.15, 4.03 ($C\mathit{H}_3$), around 4.15 to 4.60 (protons of piperidine ring), around 4.80 to 5.15 (protons at 25-position and 28-position), around 5.85 to 6.15, 1.63, 6.86, 6.97, 7.78 (protons at 17-position, 19-position and 29-position and protons of benzoxazine ring), 7.68 (amide proton) and 15.08 (phenolic proton)

Visible absorption spectrum [λ max. nm ($E^{1\%}_{1\,cm}$)] 365 (164), 482 (50) and 648 (515)

Elementary analysis for $C_{50}H_{61}N_3O_{12}$

Calcd. (%): C 67.02, H 6.86, N 4.69
Found (%): C 67.12, H 6.74, N 4.55

Example 28

[Synthesis of derivative No. 28]

A solution of 1.0 g of benzoxazinorifamycin in 5 ml of DMSO was mixed with 0.26 g of 3-hydroxypiperidine and 1.0 g of manganese dioxide and the mixture was stirred at room temperature for 4 hours. After completion of the reaction, the procedure of Example 2 was repeated to give 0.65 g of the desired derivative No. 28.

Example 29

[Synthesis of derivative No. 29]

A solution of 2.0 g of benzoxazinorifamycin in 25 ml of methanol was mixed with 2.58 g of 4-hydroxypiperidine and 4.25 ml of a solution of 1.2 N hydrochloric acid in methanol and the mixture was stirred at room temperature for 6.5 hours. After completion of the reaction, the procedure of Example 1 was repeated to give 0.92 g of the desired derivative No. 29.

Example 30

[Synthesis of derivative No. 30]

A solution of 2.0 g of benzoxazinorifamycin in 25 ml of methanol was mixed with 3.27 g of nipecotamide and 4.25 ml of a solution of 1.2 N hydrochloric acid in methanol and the mixture was stirred at room temperature for 6.5 hours. After completion of the reaction, the procedure of Example 1 was repeated to give 0.86 g of the desired derivative No. 30.

Example 31

[Synthesis of derivative No. 31]

A solution of 1.0 g of benzoxazinorifamycin in 5 ml of DMSO was mixed with 4.7 g of N,N-diethylnipecotamide and 1.0 g of manganese dioxide and the mixture was stirred at room temperature for 7 hours. After completion of the reaction, the procedure of Example 2 was repeated to give 0.33 g of the desired derivative No. 31.

Example 32

[Synthesis of derivative No. 32]

A solution of 1.0 g of benzoxazinorifamycin in 5 ml of DMSO was mixed with 0.45 ml of 4-benzylpiperidine and 1.0 g of manganese dioxide and the mixture was stirred at room temperature for 2 hours. After completion of the reaction, the procedure of Example 2 was repeated to give 0.72 g of the desired derivative No. 32.

Example 33

[Synthesis of derivative No. 33]

A solution of 1.0 g of benzoxazinorifamycinin 5 ml of DMSO was mixed with 0.29 g of 2-piperidinemethanol and 1.0 g of manganese dioxide and the mixture was stirred at room temperature for 11 days. After completion of the reaction, the procedure of Example 2 was repeated to give 0.25 g of the desired derivative No. 33.

Example 34

[Synthesis of derivative No. 34]

A solution of 1.0 g of benzoxazinorifamycin in in 5 ml of DMSO was mixed with 0.43 g of 4-piperidinopiperidine and 1.0 g of manganese dioxide and the mixture was stirred at room temperature for 4 hours. After completion of the reaction, the procedure of Example 2 was repeated to give 0.48 g of the desired derivative No. 34.

Example 35

[Synthesis of derivative No. 35]

A solution of 1.0 g of benzoxazinorifamycin in 5 ml of DMSO was mixed with 0.33 ml of 1,4-dioxa-8-azspiro[4.5]decane and 1.0 g of manganese dioxide and the mixture was stirred at room temperature for 2.5 hours. After completion of the reaction, the procedure of Example 2 was repeated to give 0.17 g of the desired derivative No. 35.

Example 36

[Synthesis of derivative No. 36]

A solution of 5.11 g of benzoxazinorifamycin in 65 ml of methanol was mixed with 10 g of 4-piperidon monohydrate hydrochloride and 7.26 ml of triethylamine and the mixture was stirred at room temperature for 6.5 hours. After completion of the reaction, the procedure of Example 1 was repeated to give 0.33 g of the desired derivative No. 36.

Example 37

[Synthesis of derivative No. 37]

A solution of 2.0 g of benzoxazinorifamycin in 25 ml of methanol was mixed with 2.33 ml of 1,2,3,6-tetrahydropyridine and 4.25 ml of a solution 1.2 N hydrochloric acid in methanol and the mixture was stirred at room temperature for 6.5 hours. After completion of the reaction, the procedure of Example 1 was repeated to give 0.38 g of the desired derivative No. 37.

Example 38

[Synthesis of derivative No. 38]

A solution of 1.0 g of benzoxazinorifamycin in 5 ml of DMSO was mixed with 0.32 ml of 1,2,3,4-tetrahydroisoquinoline and 1.0 g of manganese dioxide and the mixture was stirred over night at room temperature. After completion of the reaction, the procedure of Example 2 was repeated to give 0.59 g of the desired derivative No. 38.

Example 39

[Synthesis of derivative No. 39]

A solution of 2.0 g of benzoxazinorifamycin in 20 ml of methanol was mixed with 2.5 g of N-methylpiperazine and 4.2 ml of a solution of 1.2 N hydrochloric acid in methanol and the mixture was stirred at room temperature for 17 hours. After completion of the reaction, the procedure of Example 19 was repeated to give 0.98 g of the desired derivative No. 39.

Infrared absorption spectrum ($\nu$ $cm^{-1}$): 3450, 2970, 2940, 1720, 1660, 1600, 1560, 1480, 1460, 1420, 1400, 1370, 1300, 1250, 1220, 1170, 1130, 1080, 1040, 1020, 1000, 980, 950, 920, 900, 820, 770, 740, 700, 640, 610 and 510

Rf = 0.32, dark blue spot [chloroform-methanol (90:10)]

Nuclear magnetic resonance spectrum δ (ppm) ($CDCl_3$): 0.50, 0.72, 0.91 ($CHCH_3$), 1.80, 2.03, 2.11, 2.20, 2.23, 2.34, 2.63 and 3.03 ($CH_3$ or protons of piperazine ring), around 4.8 to 5.1 (protons at 25-position and 28-position), around 5.9 to 7.0 (protons at 17-position, 19-position and 29-position and protons of benzoxazine ring), 7.8 (proton of benzoxazine ring) and 14.95 (phenolic proton)

Visible absorption spectrum [λ max. nm ($E^{1\%}_{1cm}$)] 357 (140), 482 (76) and 620 (378)

Elementary analysis for $C_{48}H_{54}N_8O_{12}$

Calcd. (%): C 65.29, H 6.62, N 6.35
Found (%): C 65.19, H 6.49, N 6.48

Example 40

[Synthesis of derivative No. 40]

A solution of 1.0 g of benzoxazinorifamycin in 5 ml of DMSO was mixed with 0.29 g of 2,6-dimethylpiperazine and 1.0 g of manganese dioxide and the mixture was stirred at room temperature for 4 hours. After completion of the reaction, the procedure of Example 2 was repeated to give 0.55 g of the desired derivative No. 40.

Example 41

[Synthesis of derivative No. 41]

A solution of 1.0 g of benzoxazinorifamycin in 5 ml of DMSO was mixed with 0.48 ml of 1-[3-(trifluoromethyl)phenyl]piperazine and 1.0 g of manganese dioxide and the mixture was stirred at room temperature for 2 hours. After completion of the reaction, the procedure of Example 2 was repeated to give 0.49 g of the desired derivative No. 41.

Example 42

[Synthesis of derivative No. 42]

A solution of 1.0 g of benzoxazinorifamycin in 5 ml of DMSO was mixed with 0.56 g of 1-piperonylpiperazine and 1.0 g of manganese dioxide and the mixture was stirred at room temperature for 4.5 hours. After completion of the reaction, the procedure of Example 2 was repeated to give 0.74 g of the desired derivative No. 42.

Example 43

[Synthesis of derivative No. 43]

A solution of 1.57 g of benzoxazinorifamycin in 20 ml of methanol was mixed with 1.74 ml of morpholine and 3.3 ml of a solution of 1.2 N hydrochloric acid in methanol and the mixture was stirred at room temperature for 6.5 hours. After completion of the reaction, the procedure of Example 1 was repeated to give 0.39 g of the desired derivative No. 43.

Example 44

[Synthesis of derivative No. 44]

A solution of 1.0 g of benzoxazinorifamycin in 5 ml of DMSO was mixed with 0.32 ml of 2,6-dimethylmorpholine and 1.0 g of manganese dioxide and the mixture was stirred at room temperature for 4.5 hours. After completion, of the reaction, the procedure of Example 2 was repeated to give 0.20 g of the desired derivative No. 44.

Example 45

[Synthesis of derivative No. 45]

A solution of 1.0 g of benzoxazinorifamycin in 5 ml of DMSO was mixed with 0.26 ml of thiomorpholine and 1.0 g of manganese dioxide and the mixture was stirred at room temperature for 5 hours. After completion of the reaction, the procedure of Example 2 was repeated to give 0.88 g of the desired derivative No. 45.

Example 46

[Synthesis of Benzoxazinorifamycin derivative represented by the formula (IV)]

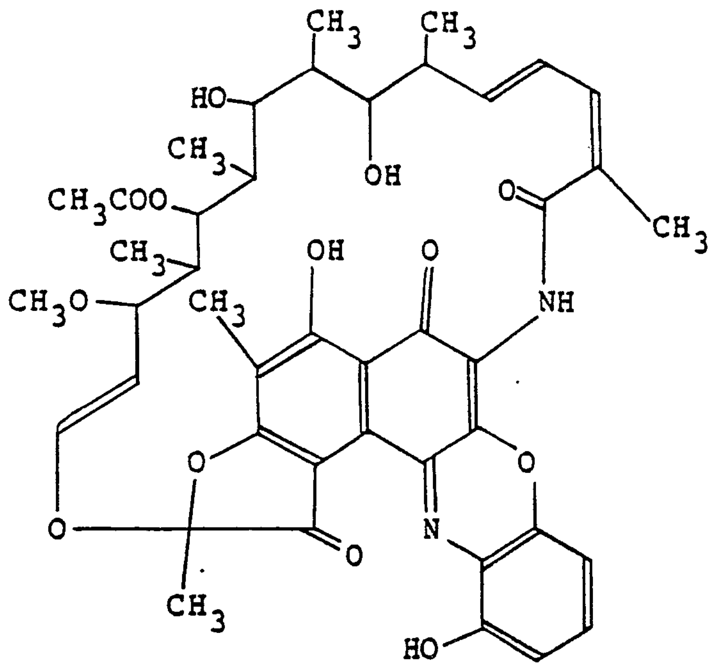

(IV)

A mixture of 2.42 g of 2-aminoresorcinol hydrochloride, 2.24 ml of triethylamine and 5 ml of chloroform was added 11 times at a thirty to forty minutes' interval into a solution of 34.79 g of rifamycin S in 200 ml of chloroform while stirring the mixture. Both thirty minutes after the eighth addition of 2-aminoresorcinol and thirty minutes after the last addition of 2-aminoresorcinol, 30 g of manganese dioxide was added to the mixture. After completion of the reaction the procedure of Example 2 was repeated to give 3.17 g of benzoxazinorifamycin derivative having the formula (IV).

Infrared absorption spectrum (ν $cm^{-1}$): 3450, 3260, 2970, 2930, 2880, 1700, 1660, 1615, 1598, 1565, 1525, 1495, 1455, 1420, 1370, 1340, 1310, 1285, 1250, 1210, 1180, 1143, 1063, 1050, 1008, 975, 945, 908, 895, 810, 790, 770, 755, 660, 630 and 440

Nuclear magnetic resonance spectrum δ (ppm) ($CDCl_3$): 0.40, 0.77, 0.93 ($CHC\mathit{H}_3$), 1.83, 2.02, 2.12, 2.27, 3.05 ($CH_3$), around 4.80 to 5.10 (protons at 25-position and 28-position), around 5.5 to 6.5 (protons at 17-position, 19-position and 29-position), 6.77, 6.90, 7.43 (protons of benzoxazine ring), 7.93 (amide proton), 9.92 and 14.33 (phenolic protons).

Example 47

[Synthesis of derivative No. 46]

A solution of 0.8 g of the derivative represented by the formula (IV) prepared in Example 46 in 10 ml of DMSO was mixed with 0.15 g of pyrrolidine and 0.8 g of manganese dioxide and the mixture was stirred over night at room temperature. After completion of the reaction, the procedure of Example 2 was repeated to give 0.10 g of the desired derivative No. 46.

Example 48

[Synthesis of derivative No. 47]

A solution of 0.8 g of the derivative represented by the formula (IV) prepared in Example 46 in 10 ml of DMSO was mixed with 0.17 g of piperidine and 0.8 g of manganese dioxide and the mixture was stirred over night at room temperature. After completion of the reaction, the procedure of Example 2 was repeated to give 0.37 g of the desired derivative No. 47.

Example 49

[Synthesis of derivative No. 48]

A solution of 0.8 g of the derivative represented by the formula (IV) prepared in Example 46 in 10 ml of DMSO was mixed with 0.2 g of N-methylpiperazine and 0.8 g of manganese dioxide and the mixture was stirred at room temperature for two days. After completion of the reaction, the procedure of Example 2 was repeated to give 0.44 g of the desired derivative No. 48.

Example 50

[Synthesis of derivative No. 49]

One gram of the derivative No. 18 prepared in Example 18 was added to a mixture of water and ethanol (1:1) containing 0.96 g of sodium hydroxide and the mixture was stirred at room temperature for 1.5 hours. After completion of the reaction, the reaction mixture was diluted with cold water, which was then neutralized with 1N hydrochloric acid solution and extracted with chloroform. The extract was evaporated to dryness under reduced pressure and the procedure of Example 2 was repeated to give 0.63 g of the desired derivative No. 49.

Infrared absorption spectrum (ν $cm^{-1}$): 3430, 2960, 2930, 2880, 1710, 1672, 1601, 1485, 1465, 1398, 1378, 1350, 1320, 1285, 1265, 1235, 1175, 1150, 1135, 1120, 1060, 1040, 980, 950, 910, 860, 825, 775, 660, 600 and 435

Nuclear magnetic resonance spectrum δ (ppm) ($CDCl_3$): 0.83, 0.88 0.93 ($CHCH_3$), 1.83, 2.04, 2.18, 3.02 ($CH_3$), 2.85 to 3.85 (protons of pyrrolidine ring), around 4.90 to 5.30 (protons at 25-position and 28-position), 5.64, 5.77, around 6.00 to 6.50, 6.85 (protons at 17-position, 19-position and 29-position and protons of benzoxazine ring), 71.5 (amide proton) and 15.62 (phenolic proton)

Visible absorption spectrum [λ max. nm ($E^{1\%}_{1cm}$)]: 363 (167), 478 (46) and 642 (516)

Elementary analysis for $C_{45}H_{53}N_3O_{11}$

Calcd. (%): C 66.57, H 6.58, N 5.17

Found (%): C 66.70, H 6.78, N 4.99

Example 51

[Synthesis of derivative No. 50]

The procedure of Example 50 was repeated except that 1.0 g of the derivative No. 19 was employed in place of the derivative No. 18 to give 0.59 g of the desired derivative No. 50.

Example 52

[Synthesis of derivative No. 51]

The procedure of Example 50 was repeated except that 0.22 g of the derivative No. 39 was employed in place of the derivative No. 18 to give 0.13 g of the desired derivative No. 51.

## Claims

1. A rifamycin derivative having the general formula (I):

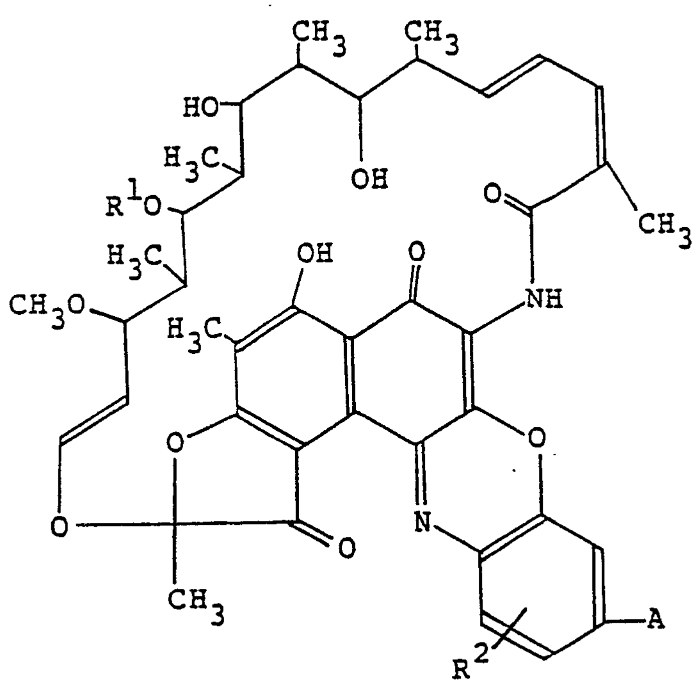

(I)

wherein $R^1$ is hydrogen atom or acetyl group; $R^2$ is hydrogen atom or hydroxyl group; A is a group represented by the formula:

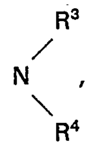,

wherein $R^3$ is an alkyl group with 1 to 5 carbon atoms or an alkoxyalkyl group with 2 to 6 carbon atoms and $R^4$ is an alkyl group with 1 to 5 carbon atoms, a group represented by the formula: $-(CH_2)_aX^1$, wherein a is 1 to 4 and $X^1$ is ethynyl group, cyano group, a group having the formula:

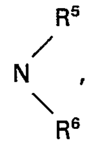,

wherein $R^5$ and $R^6$ are the same or different from each other and are hydrogen atom or an alkyl group with 1 to 3 carbon atoms, a group represented by the formula: $OR^7$, wherein $R^7$ is hydrogen atom or an alkyl group with 1 to 4 carbon atoms, a group represented by the formula:

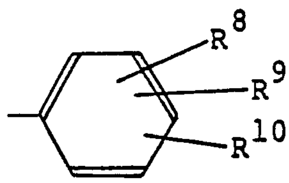,

wherein $R^8$, $R^9$ and $R^{10}$ are the same or different from each other and are hydrogen atom or an alkoxy group with 1 to 3 carbon atoms, or a group represented by the formula:

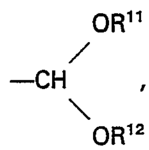,

wherein $R^{11}$ and $R^{12}$ are the same or different from each other and are an alkyl group with 1 to 3 carbon atoms; a cycloalkyl group with 3 to 8 carbon atoms, a group represented by the formula:

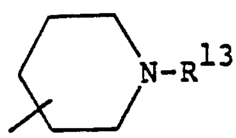,

wherein $R^{13}$ is hydrogen atom or an alkyl group with 1 to 3 carbon atoms, or a group represented by the formula: $-CH_2(CHOH)_4CH_2OH$;
a group represented by the formula:

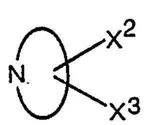,

wherein

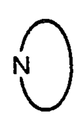

is a 3 to 10 membered cyclic amino group with 2 to 9 carbon atoms, $X^2$ is hydrogen atom or an alkyl group having 1 to 4 carbon atoms and $X^3$ is hydrogen atom, an alkyl group with 1 to 3 carbon atoms, hydroxyl group, a group represented by the formula:

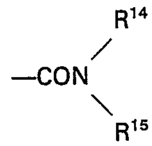

,

wherein $R^{14}$ and $R^{15}$ are the same or different from each other and are hydrogen atom or an alkyl group with 1 to 4 carbon atoms, a hydroxyalkyl group with 1 to 3 carbon atoms or cyclic or noncyclic amino group with 2 to 6 carbon atoms, or $X^2$ and $X^3$ when taken together represent = O group or the group having the formula: $-O(CH_2)_bO-$, wherein b is 2 to 4, a group represented by the formula:

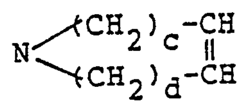

,

wherein c and d are the same or different from each other and are 1 to 4, a group represented by the formula:

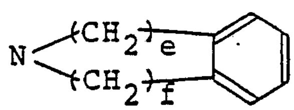

,

wherein e and f are the same of different from each other and are 1 to 4, or a group represented by the formula:

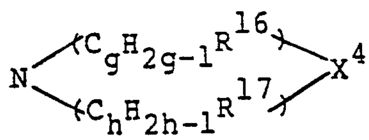

,

wherein g and h are the same or different from each other and are 1 to 4, $R^{16}$ and $R^{17}$ are the same or different from each other and are hydrogen atom or an alkyl group with 1 to 3 carbon atoms and $X^4$ is oxygen atom, sulfur atom or a group represented by the formula: $NR^{18}$, wherein $R^{18}$ is hydrogen atom, an alkyl group with 1 to 3 carbon atoms, a phenyl group represented by the formula:

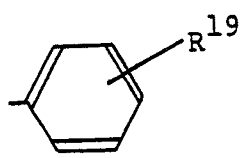

,

wherein $R^{19}$ is hydrogen atom or trifluoromethyl group, or a group represented by the formula:

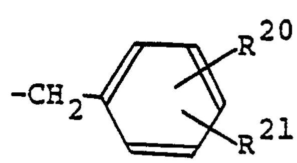

,

wherein $R^{20}$ and $R^{21}$ are hydrogen atoms or when taken together represent the group having the formula $-OCH_2O-$, or salts thereof.

2. The rifamycin derivative or salts thereof of Claim 1, wherein $R^1$ is acetyl group in the general formula (I).

3. The rifamycin derivative or salts thereof of Claim 1, wherein $R^1$ is hydrogen atom in the general formula (I).

4. The rifamycin derivative or salts thereof of Claim 1, 2 or 3, wherein $R^2$ is hydrogen atom in the general formula (I).

5. The rifamycin derivative or salts thereof of Claim 1, 2 or 3, wherein $R^2$ is hydroxyl group in the general formula (I).

6. The rifamycin derivative or salts thereof of Claim 1, 2 or 4, wherin $R^1$ is acetyl group and $R^2$ is hydrogen atom in the general formula (I).

7. The rifamycin derivative or salts thereof of Claim 1, 3 or 4, wherein both $R^1$ and $R^2$ are hydrogen atoms in the general formula (I).

8. The rifamycin derivative or salts thereof of Claim 1, 2, 3, 4, 5, 6 or 7, wherein A is a group represented by the formula:

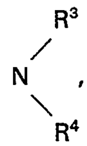 ,

wherein $R^3$ is an alkyl group with 1 to 5 carbon atoms or an alkoxyalkyl group with 2 to 6 carbon atoms and $R^4$ is an alkyl group with 1 to 5 carbon atoms, a group represented by the formula: $-(CH_2)_aX^1$, wherein a is 1 to 4 and $X^1$ is ethynyl group, cyano group, a group represented by the formula:

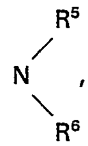 ,

wherein $R^5$ and $R^6$ are the same or different from each other and are hydrogen atom or an alkyl group with 1 to 3 carbon atoms, a group represented by the formula: $OR^7$, wherein $R^7$ is a hydrogen atom or a alkyl group with 1 to 4 carbon atoms, a group represented by the formula:

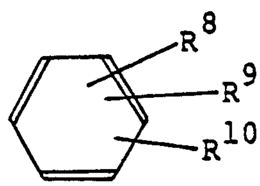 ,

wherein $R^8$, $R^9$ and $R^{10}$ are the same or different from each other and are hydrogen atom or an alkoxy group with 1 to 3 carbon atoms, or a group represented by the formula:

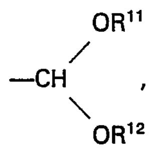 ,

wherein $R^{11}$ and $R^{12}$ are the same or different from each other and are an alkyl group with 1 to 3 carbon atoms; a cycloalkyl group with 3 to 8 carbon atoms, a group represented by the formula:

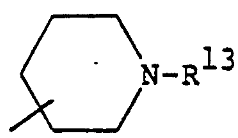 ,

wherein $R^{13}$ is hydrogen atom or an alkyl group with 1 to 3 carbon atoms, or a group represented by the formula: $-CH_2(CHOH)_4CH_2OH$ in the general formula (I).

9. The rifamycin derivative or salts thereof of Claim 1, 2, 3, 4, 5, 6 or 7, wherein A is a group represented by the formula:

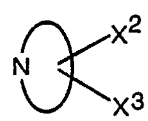 ,

wherein

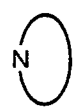

is a 3 to 10 membered cyclic amino group with 2 to 9 carbon atoms, $X^2$ is hydrogen atom or an alkyl group with 1 to 4 carbon atoms and $X^3$ is hydrogen atom, an alkyl group with 1 to 3 carbon atoms, hydroxyl group, a group represented by the formula:

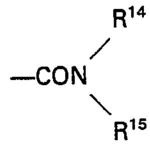
,

wherein $R^{14}$ and $R^{15}$ are the same or different from each other and are hydrogen atom or an alkyl group with 1 to 4 carbon atoms, a hydroxyalkyl group with 1 to 3 carbon atoms or cyclic or noncyclic amino group with 2 to 6 carbon atoms, or $X^2$ and $X^3$ when taken together represent = O group or the group having the formula: $-O(CH_2)_bO-$, wherein b is 2 to 4 in the general formula (I).

10. The rifamycin derivative or salts thereof of Claim 1, 2, 3, 4, 5, 6 or 7, wherein A is a group represented by the formula:

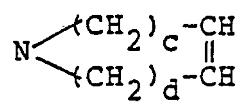
,

wherein c and d are the same or different from each other and are 1 to 4, or a group represented by the formula:

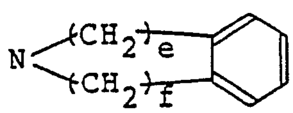
,

wherein e and f are the same or different from each other and are 1 to 4 in the general formula (I).

11. The rifamycin derivative or salts thereof of Claim 1, 2, 3, 4, 5, 6 or 7, wherein A is a group represented by the formula:

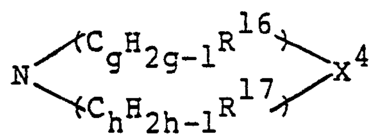
,

wherein g and h are the same or different from each other and are 1 to 4, $R^{16}$ and $R^{17}$ are the same or different from each other and are hydrogen atom or an alkyl group with 1 to 3 carbon atoms and $X^4$ is oxygen atom, sulfur atom or a group represented by the formula: $NR^{18}$, wherein $R^{18}$ is hydrogen atom, an alkyl group with 1 to 3 carbon atoms, a phenyl group represented by the formula:

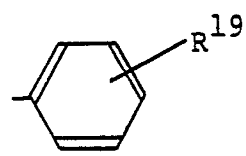
,

wherein $R^{19}$ is hydrogen atom or trifluoromethyl group, or a group represented by the formula:

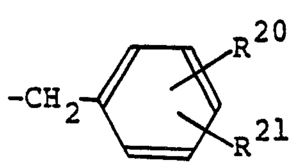
,

wherein $R^{20}$ and $R^{21}$ are hydrogen atoms or when taken together represent the group having the formula: $-OCH_2O-$ in the general formula (I).

12. The rifamycin derivative or salts thereof of Claim 1, wherein $R^1$ is acetyl group, $R^2$ is hydrogen atom, $R^3$ is methyl group and $R^4$ is ethyl group in the general formula (I).

13. The rifamycin derivative or salts thereof of Claim 1, wherein $R^1$ is acetyl group, $R^2$ is hydrogen atom

and both $R^3$ and $R^4$ are ethyl groups in the general formula (I).

14. The rifamycin derivative or salts thereof of Claim 1, wherein $R^1$ is acetyl group, $R^2$ is hydrogen atom and both $R^3$ and $R^4$ are propyl groups in the general formula (I).

15. The rifamycin derivative or salts thereof of Claim 1, wherein $R^1$ is acetyl group, $R^2$ is hydrogen atom and both $R^3$ and $R^4$ are ethoxyethyl groups in the general formula (I).

16. The rifamycin derivative or salts thereof of Claim 1, wherein $R^1$ is acetyl group, $R^2$ is hydrogen atom, $R^3$ is methyl group and $R^4$ is 2,2-dimethoxyethyl group in the general formula (I).

17. The rifamycin derivative or salts thereof of Claim 1, wherein $R^1$ is acetyl group, $R^2$ is hydrogen atom and A is a group represented by the formula:

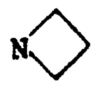

in the general formula (I).

18. The rifamycin derivative or salts thereof of Claim 1, wherein $R^1$ is acetyl group, $R^2$ is hydrogen atom and A is a group represented by the formula:

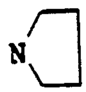

in the general formula (I).

19. The rifamycin derivative or salts thereof of Claim 1, wherein $R^1$ is acetyl group, $R^2$ is hydrogen atom and A is a group represented by the formula:

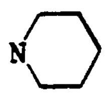

in the general formula (I).

20. The rifamycin derivative or salts thereof of Claim 1, wherein $R^1$ is acetyl group, $R^2$ is hydrogen atom and A is a group represented by the formula:

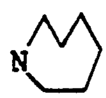

in the general formula (I).

21. The rifamycin derivative or salts thereof of Claim 1, wherein $R^1$ is acetyl group, $R^2$ is hydrogen atom and A is a group represented by the formula:

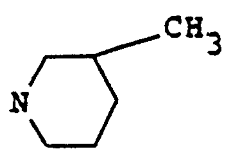

in the general formula (I).

22. The rifamycin derivative or salts thereof of Claim 1, wherein $R^1$ is acetyl group, $R^2$ is hydrogen atom and A is a group represented by the formula:

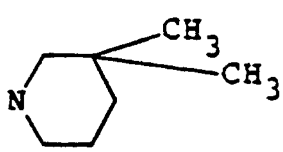

in the general formula (I).

23. The rifamycin derivative or salts thereof of Claim 1, wherein $R^1$ is hydrogen atom, $R^2$ is hydrogen atom and A is a group represented by the formula:

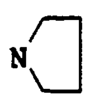

in the general formula (I).

24. A process for preparing a rifamycin derivative having the general formula (I):

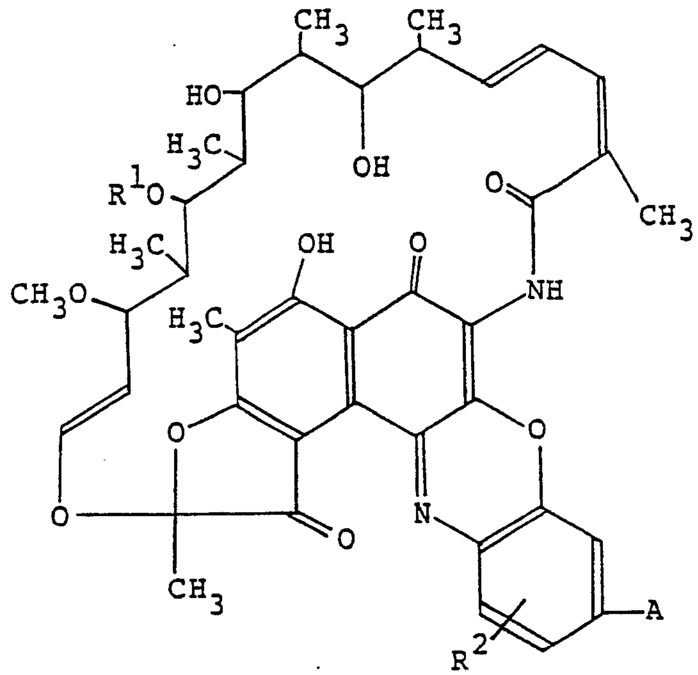

(I)

wherein $R^1$ is hydrogen atom or acetyl group; $R^2$ is hydrogen atom or hydroxyl group; A is as defined in claim 1, or salts thereof, which comprises reacting a rifamycin derivative having the general formula (II):

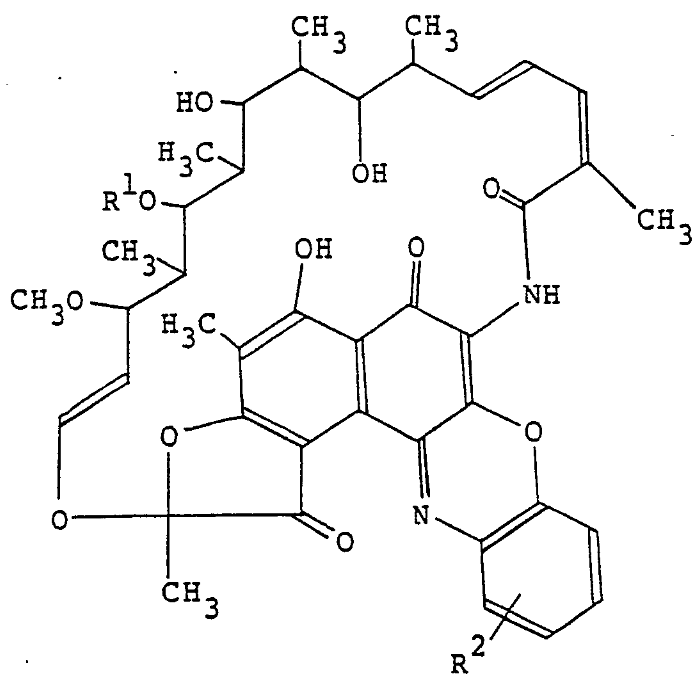

(II)

wherein $R^1$ and $R^2$ are as defined above, with an amine having the general formula: AH, wherein A is as defined above.

25. The process of Claim 24, wherein the rifamycin derivative having the general formula (II) is reacted with the amine having the general formula: AH in the presence of an oxydizing agent.

26. Process of Claim 24 or 25, wherein the oxydizing agent is manganese dioxide.

27. A process for preparing a rifamycin derivative having the general formula (I), wherein $R^1$ is hydrogen atom and $R^2$ and A are as defined above, which comprises hydrolizing a rifamycin derivative having the general formula (I), wherein $R^1$ is acetyl group, and $R^2$ and A are as above.

28. The process of Claim 27, wherein an agent employed for hydrolysis is alkali metal hydroxide.

29. Antibacterial agents containing a rifamycin derivative having the general formula (I):

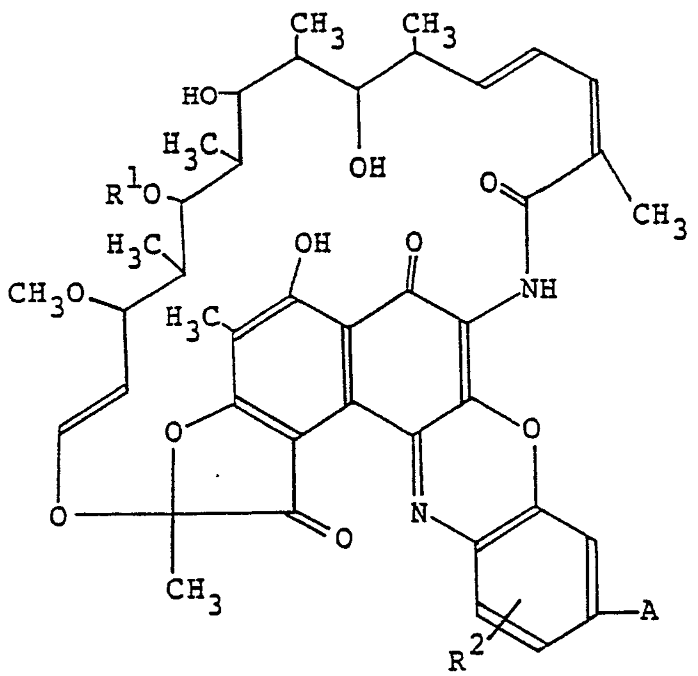

(I)

wherein $R^1$ is hydrogen atom or acetyl group; $R^2$ is hydrogen atom or hydroxyl group; A is as defined in claim 1, or salts thereof as an effective component.

**Patentansprüche**

1. Rifamycinderivat mit der allgemeinen formel (I):

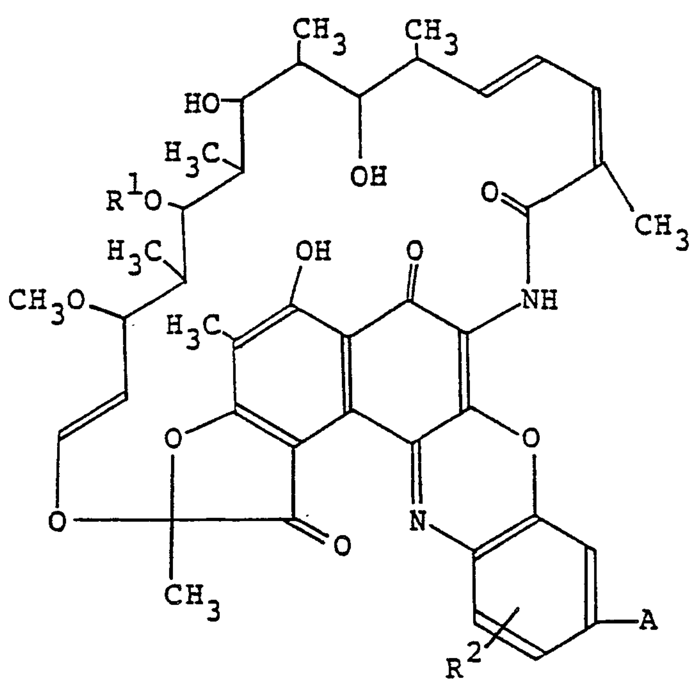

(I)

worin $R^1$ Wasserstoffatom oder Acetylgruppe ist; $R^2$ Wasserstoffatom oder Hydroxylgruppe ist; A eine Gruppe ist dargestellt durch die Formel:

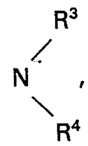,

worin $R^3$ eine Alkylgruppe mit 1 bis 5 Kohlenstoffatomen oder eine Alkoxyalkylgruppe mit 2 bis 6 Kohlenstoffatomen ist und $R^4$ eine Alkylgruppe mit 1 bis 5 Kohlenstoffatomen, eine Gruppe dargestellt durch die Formel: $-(CH_2)_aX^1$, worin a für 1 bis 4 steht und $X^1$ ist Ethynylgruppe, Cyanogruppe, eine Gruppe mit der Formel:

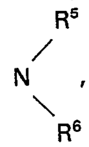,

worin $R^5$ und $R^6$ gleich oder verschieden voneinander sind und sind Wasserstoffatom oder eine Alkylgruppe mit 1 bis 3 Kohlenstoffatomen, eine Gruppe dargestellt durch die Formel: $OR^7$, worin $R^7$ ist Wasserstoffatom oder eine Alkylgruppe mit 1 bis 4 Kohlenstoffatomen, eine Gruppe dargestellt durch die Formel:

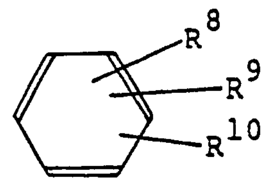

worin $R^8$, $R^9$ und $R^{10}$ gleich oder verschieden voneinander sind und sind Wasserstofatom oder eine Alkoxygruppe mit 1 bis 3 kohlenstoffatomen oder eine Gruppe dargestellt durch die Formel:

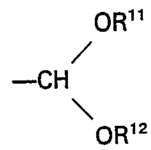

worin $R^{11}$ und $R^{12}$ gleich oder verschieden voneinander sind und eine Alkylgruppe mit 1 bis 3 Kohlenstoffatomen; eine Cycloalkylgruppe mit 3 bis 8 Kohlenstoffatomen, eine Gruppe dargestellt durch die Formel:

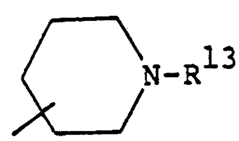

worin $R^{13}$ ist Wasserstoffatom oder eine Alkylgruppe mit 1 bis 3 Kohlenstoffatomen oder eine Gruppe dargestellt durch die Formel: $-CH_2(CHOH)_4CH_2OH$; eine Gruppe dargestellt durch die Formel:

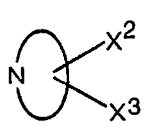,

worin

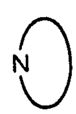

ist eine 3- bis 10-gliedrige cyclische Aminogruppe mit 2 bis 9 Kohlenstoffatomen, $X^2$ is Wasserstoffatom oder eine Alkylgruppe mit 1 bis 4 Kohlenstoffatomen und $X^3$ ist Wasserstoffatom, eine Alkylgruppe mit 1 bis 3 Kohlenstoffatomen, Hydroxylgruppe, eine Gruppe dargestellt durch die Formel:

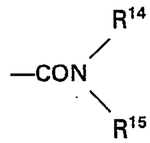

,

worin $R^{14}$ und $R^{15}$ gleich oder verschieden voneinander sind und sind Wasserstoffatom oder eine Alkylgruppe mit 1 bis 4 Kohlenstoffatomen, eine Hydroxyalkylgruppe mit 1 bis 3 Kohlenstoffatomen oder cyclische oder nicht-cyclische Aminogruppe mit 2 bis 6 Kohlenstoffatomen, oder $X^2$ und $X^3$, wenn zusammengenomnen =O-Gruppe darstellen oder die Gruppe mit der Formel: $—O(CH_2)_bO—$, worin b 2 bis 4 ist, eine Gruppe dargestellt durch die Formel:

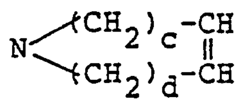

,

worin c und d gleich oder verschieden voneinander sind und 1 bis 4 sind, eine Gruppe dargestellt durch die Formel:

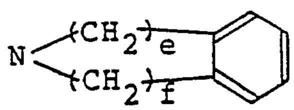

,

worin e und f gleich oder verschieden voneinander sind und 1 bis 4 sind, oder eine Gruppe dargestellt durch die Formel:

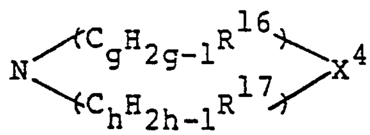

worin g und h gleich oder verschieden voneinander sind und 1 bis 4 sind, $R^{16}$ und $R^{17}$ gleich oder verschieden voneinander sind und sind Wasserstoffatom oder eine Alkylgruppe mit 1 bis 3 Kohlenstoffatomen und $X^4$ ist Sauerstoffatom, Schwefelatom oder eine Gruppe, dargestellt durch die Formel: $NR^{18}$, worin $R^{18}$ ist Wasserstoffatom, eine Alkylgruppe mit 1 bis 3 Kohlenstoffatomen, eine Phenylgruppe, dargestellt durch die Formel:

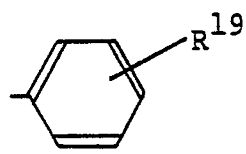

,

worin $R^{19}$ ist Wasserstoffatom oder Trifluoromethylgruppe, oder eine Gruppe dargestellt durch die Formel:

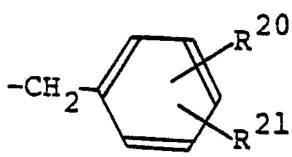

,

worin $R^{20}$ und $R^{21}$ sind Wasserstoffatome oder, wenn zusammengenommen, die Gruppe mit der Formel: $—OCH_2O—$ darstellen, oder Salze davon.

2. Rifamycinderivat oder Salze davon nach Anspruch 1, worin in der allgemeinen formel (I) $R^1$ Acetylgruppe ist.

3. Rifamycinderivat oder Salze davon nach Anspruch 1, worin in der allgemeinen Formel (I) $R^1$ Wasserstoffatom ist.

4. Rifamycinderivat oder Salze davon nach Anspruch 1, 2 oder 3, worin in der allgemeinen formel (I) $R^2$ Wasserstoffatom ist.

5. Rifamycinderivat oder Salze davon nach Anspruch 1, 2 oder 3, worin in der allgemeinen Formel (I) $R^2$ Hydroxylgruppe ist.

6. Rifamycinderivat oder Salze davon nach Anspruch 1, 2 oder 4, worin in der allgemeinen Formel (I) $R^1$ Acetylgruppe ist und $R^2$ Wasserstoffatom ist.

7. Rifamycinderivat oder Salze davon nach Anspruch 1, 3 oder 4, worin in der allgemeinen Formel (I) beide, $R^1$ und $R^2$, Wasserstoffatome sind.

8. Rifamycinderivat oder Salze davon nach Anspruch 1, 2, 3, 4, 5, 6 oder 7, worin A eine Gruppe ist dargestellt durch die Formel:

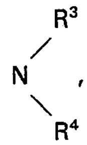 ,

worin $R^3$ eine Alkylgruppe mit 1 bis 5 Kohlenstoffatomen oder eine Alkoxyalkylgruppe mit 2 bis 6 Kohlenstoffatomen ist und $R^4$ ist eine Alkylgruppe mit 1 bis 5 Kohlenstoffatomen, eine Gruppe dargestellt durch die Formel: $—(CH_2)_aX^1$, worin a für 1 bis 4 ist und $X^1$ ist Ethynylgruppe, Cyanogruppe, eine Gruppe dargestellt durch die Formel:

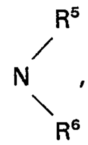 ,

worin $R^5$ und $R^6$ gleich oder verschieden voneinander sind und sind Wasserstoffatom oder eine Alkylgruppe mit 1 bis 3 Kohlenstoffatomen, eine Gruppe dargestellt durch die Formel: $OR^7$, worin $R^7$ ist Wasserstoffatom oder eine Alkylgruppe mit 1 bis 4 Kohlenstoffatomen, eine Gruppe dargestellt durch die Formel:

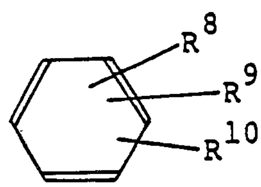

worin $R^8$, $R^9$ und $R^{10}$ gleich oder verschieden voneinander sind und sind Wasserstoffatom oder eine Alkoxygruppe mit 1 bis 3 Kohlenstoffatomen, oder eine Gruppe, dargestellt durch die Formel:

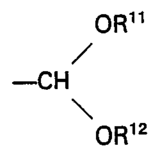

worin $R^{11}$ und $R^{12}$ gleich oder verschieden sind und eine Alkylgruppe mit 1 bis 3 Kohlenstoffatomen sind; eine Cycloalkylgruppe mit 3 bis 8 Kohlenstoffatomen, eine Gruppe dargestellt durch die Formel:

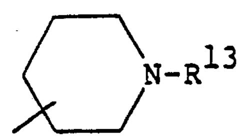

worin $R^{13}$ ist Wasserstoffatom oder eine Alkylgruppe mit 1 bis 3 Kohlenstoffatomen oder eine Gruppe dargestellt durch die Formel: $—CH_2(CHOH)_4CH_2OH$ in der allgemeinen Formel (I).

9. Rifamycinderivat oder Salze davon nach Anspruch 1, 2, 3, 4, 5, 6 oder 7, worin A eine Gruppe ist dargestellt durch die Formel:

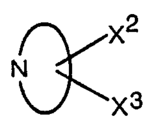 ,

worin

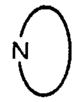

eine 3 bis 10-gliedrige cyclische Aminogruppe mit 2 bis 9 Kohlenstoffatomen, $X^2$ Wasserstoffatom oder eine Alkylgruppe mit 1 bis 4 Kohlenstoffatomen ist und $X^3$ ist Wasserstoffatom, eine Alkylgruppe mit 1 bis 3 Kohlenstoffatomen, Hydroxylgruppe, eine Gruppe dargestellt durch die Formel:

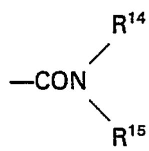 ,

worin $R^{14}$ und $R^{15}$ gleich oder verschieden voneinander sind und sind Wasserstoffatom oder eine Alkylgruppe mit 1 bis 4 Kohlenstoffatomen, eine Hydroxyalkylgruppe mit 1 bis 3 Kohlenstoffatomen oder cyclische oder nicht-cyclische Aminogruppe mit 2 bis 6 Kohlenstoffatomen, oder $X^2$ und $X^3$, wenn zusammengenommen, stellen =O-Gruppe oder die Gruppe mit der Formel: $—O(CH_2)_bO—$ dar, worin b 2 bis 4 ist, in der allgemeinen Formel (I).

10. Rifamycinderivat oder Salze davon nach Anspruch 1, 2, 3, 4, 5, 6 oder 7, worin A eine Gruppe ist dargestellt durch die Formel:

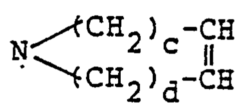 ,

worin c und d gleich oder verschieden voneinander sind und 1 bis 4 sind, eine Gruppe dargestellt durch die Formel:

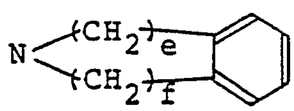 ,

worin e und f gleich oder verschieden voneinander sind und 1 bis 4 sind, in der allgemeinen Formel (I).

11. Rifamycinderivat oder Salze davon nach Anspruch 1, 2, 3, 4, 5, 6 oder 7, worin A eine Gruppe ist dargestellt durch die Formel:

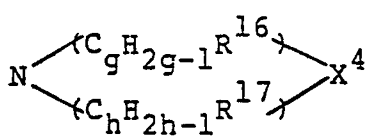 ,

worin g und h gleich oder verschieden voneinander sind und 1 bis 4 sind, $R^{16}$ und $R^{17}$ gleich oder verschieden voneinander sind und sind Wasserstoffatom oder eine Alkylgruppe mit 1 bis 3 Kohlenstoffatomen und $X^4$ ist Sauerstoffatom, Schwefelatom, eine Gruppe, dargestellt durch die Formel: $NR^{18}$, worin $R^{18}$ ist Wasserstoffatom, eine Alkylgruppe mit 1 bis 3 Kohlenstoffatomen, eine Phenylgruppe, dargestellt durch die Formel:

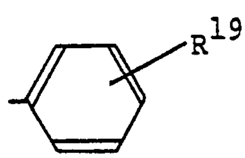 ,

worin $R^{19}$ ist Wasserstoffatom oder Trifluoromethylgruppe, oder eine Gruppe dargestellt durch die Formel:

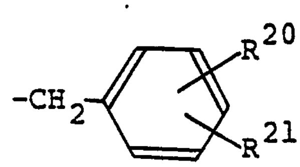 ,

worin $R^{20}$ und $R^{21}$ Wasserstoffatom sind oder, wenn zusammengenommen die Gruppe mit der Formel: $—OCH_2O—$ darstellen, in der allgemeinen Formel (I).

12. Rifamycinderivat oder Salze davon nach Anspruch 1, worin in der allgemeinen Formel (I) $R^1$ Acetylgruppe ist, $R^2$ Wasserstoffatom ist, $R^3$ Methylgruppe ist und $R^4$ Ethylgruppe ist.

13. Rifamycinderivat oder Salze davon nach Anspruch 1, worin in der allgemeinen Formel (I) $R^1$ Acetylgruppe ist, $R^2$ Wasserstoffatom ist und $R^3$ und $R^4$ beide Ethylgruppen sind.

14. Rifamycinderivat oder Salze davon nach Anspruch 1, worin in der allgemeinen Formel (I) $R^1$ Acetylgruppe ist, $R^2$ Wasserstoffatom ist und $R^3$ und $R^4$ beide Propylgruppen sind.

15. Rifamycinderivat oder Salze davon nach Anspruch 1, worin in der allgemeinen Formel (I) $R^1$ Acetylgruppe ist, $R^2$ Wasserstoffatom ist und $R^3$ und $R^4$ Ethoxyethylgruppen sind.

16. Rifamycinderivat oder Salze davon nach Anspruch 1, worin in der allgemeinen Formel (I) $R^1$ Acetylgruppe ist, $R^2$ Wasserstoffatom ist, $R^3$ Methylgruppe ist und $R^4$ 2,2-Dimethoxyethylgruppe ist.

17. Rifamycinderivat oder Salze davon nach Anspruch 1, worin in der allgemeinen Formel (I) $R^1$ Acetylgruppe ist, $R^2$ Wasserstoffatom ist und A eine Gruppe ist, dargestellt durch die Formel:

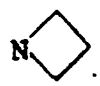

18. Rifamycinderivat oder Salze davon nach Anspruch 1, worin in der allgemeinen Formel (I) $R^1$ Acetylgruppe ist, $R^2$ Wasserstoffatom ist und A eine Gruppe ist, dargestellt durch die Formel:

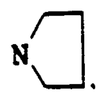

19. Rifamycinderivat oder Salze davon nach Anspruch 1, worin in der allgemeinen Formel (I) $R^1$ Acetylgruppe ist, $R^2$ Wasserstoffatom ist und A eine Gruppe ist, dargestellt durch die Formel:

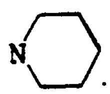

20. Rifamycinderivat oder Salze davon nach Anspruch 1, worin in der allgemeinen Formel (I) $R^1$ Acetylgruppe ist, $R^2$ Wasserstoffatom ist und A eine Gruppe ist, dargestellt durch die Formel:

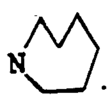

21. Rifamycinderivat oder Salze davon nach Anspruch 1, worin in der allgemeinen Formel (I) $R^1$ Acetylgruppe ist, $R^2$ Wasserstoffatom ist und A eine Gruppe ist, dargestellt durch die Formel:

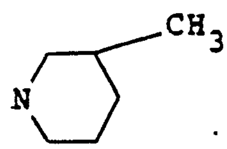

22. Rifamycinderivat oder Salze davon nach Anspruch 1, worin in der allgemeinen Formel (I) $R^1$ Acetylgruppe ist, $R^2$ Wasserstoffatom ist und A eine Gruppe ist, dargestellt durch die Formel:

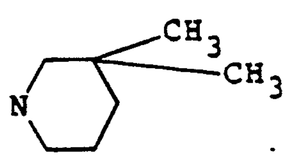

23. Rifamycinderivat oder Salze davon nach Anspruch 1, worin in der allgemeinen Formel (I) $R^1$ Wasserstoffatom ist, $R^2$ Wasserstoffatom ist und A eine Gruppe ist, dargestellt durch die Formel:

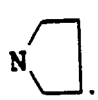

24. Verfahren zur Herstellung eines Rifamycinderivats mit der allgemeinen Formel (I):

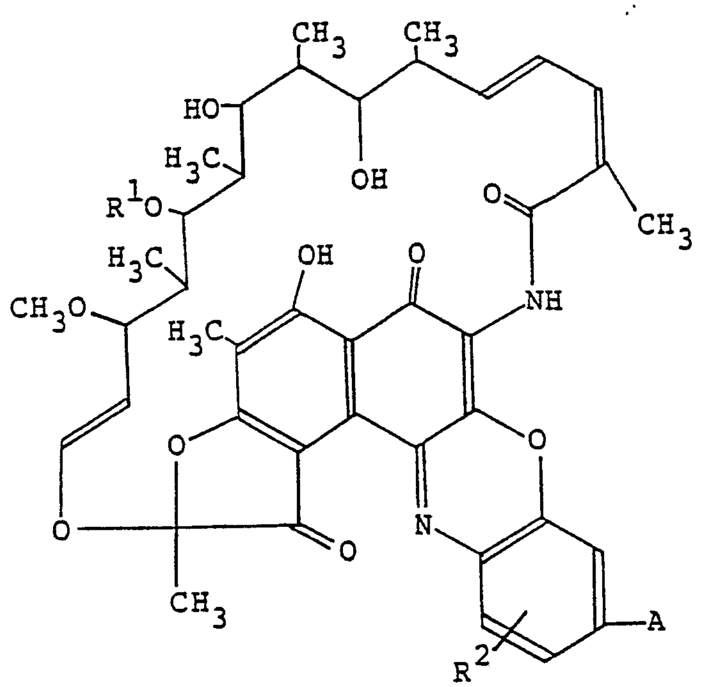

(I)

worin $R^1$ Wasserstoffatom oder Acetylgruppe ist; $R^2$ Wasserstoffatom oder Hydroxylgruppe ist; und A wie in Anspruch 1 definiert ist, oder Salze davon, welches umfaßt Umsetzen eines Rifamycinderivats mit der allgemeinen Formel (II):

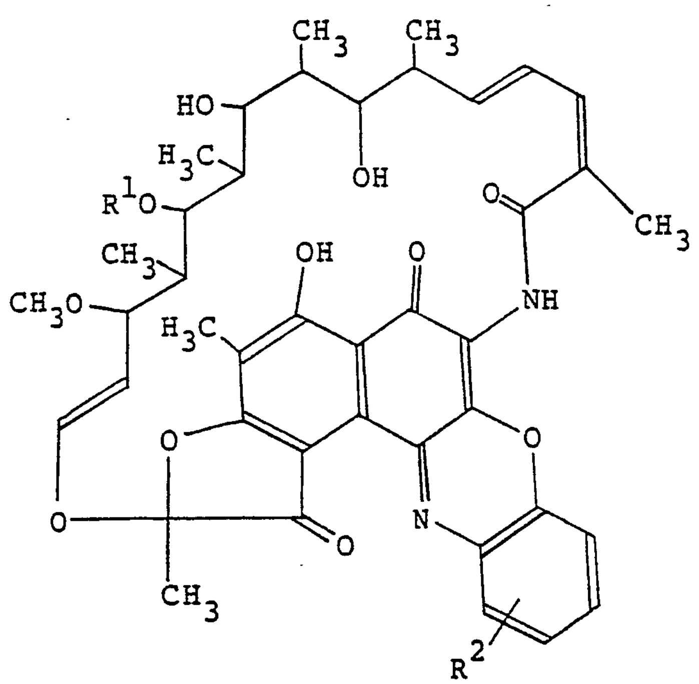

(II)

worin $R^1$ und $R^2$ wie oben definiert sind, mit einem Amin mit der allgemeinen Formel: AH, worin A wie oben definiert ist.

25. Verfahren nach Anspruch 24, worin das Rifamycinderivat mit der allgemeinen Formel (II) mit dem Amin mit der allgemeinen Formel: AH in der Gegenwart eines Oxidationsmittels umgesetzt wird.

26. Verfahren nach Anspruch 24 oder 25, worin das Oxidationsmittel Mangandioxid ist.

27. Verfahren zur Herstellung eines Rifamycinderivats mit der allgemeinen Formel (I), worin $R^1$ Wasserstoffatom ist und $R^2$ und A wie oben definiert sind, welches umfaßt Hydrol sieren eines Rifamycinderivats mit der allgemeinen Formel (I), wirin $R^1$ Acetylgruppe ist und $R^2$ und A wie oben sind.

28. Verfahren nach Anspruch 27, worin ein für die Hydrolyse verwendetes Mittel Alkalimetallhydroxid ist.

29. Antibakterielle Mittel enthaltend ein Rifamycinderivat mit der allgemeinen Formel (I):

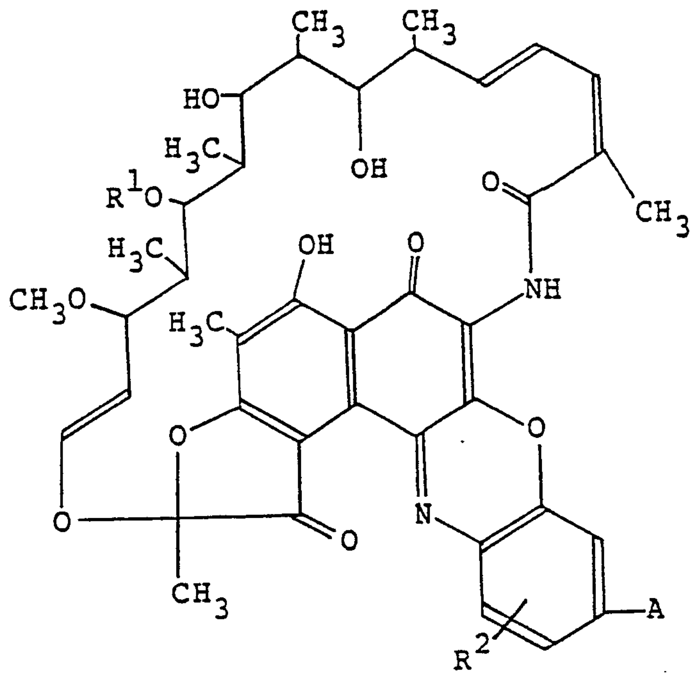

(I)

worin $R^1$ Wasserstoffatom oder Acetylgruppe ist; $R^2$ Wasserstoffatom oder Hydroxylgruppe ist; A wie in Anspruch 1 definiert ist oder Salz davon als eine wirksame Komponente.

## Revendications

1. Dérivé de rifamycine de formule générale (I):

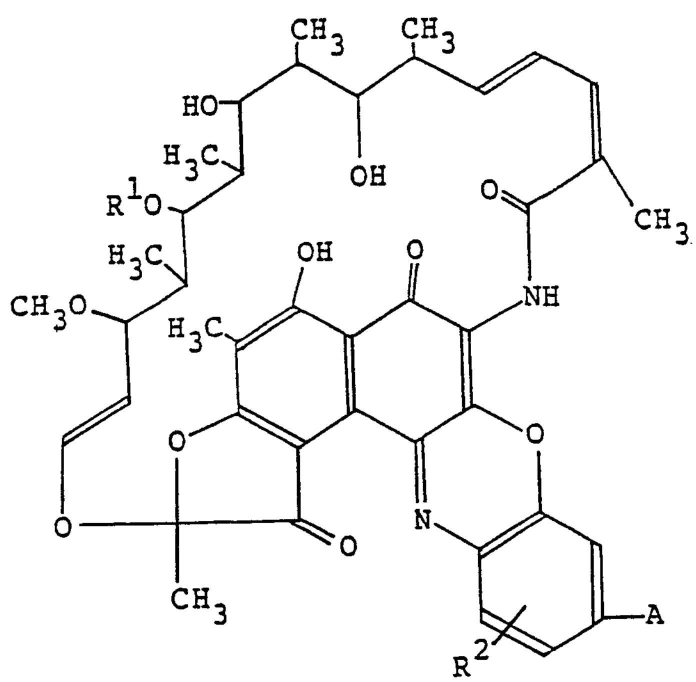

(I)

où $R^1$ est un atome d'hydrogène ou un groupe acétyle; $R^2$ est un atome d'hydrogène ou un groupe hydroxyle; A est un groupe représenté par la formula:

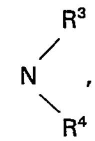
,

où $R^3$ est un groupe alcoyle en $C_1$ à $C_5$ ou un groupe alcoxyalcoyle en $C_2$ à $C_6$ et $R^4$ est un groupe alcoyle en $C_1$ à $C_5$, un groupe représenté par la formule: $-(CH_2)_aX^1$, où a vaut 1 à 4 et $X^1$ est un groupe éthynyle, un groupe cyano, un groupe de formule

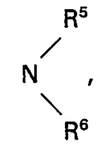
,

où $R^5$ et $R^6$ sont identiques ou différents l'un de l'autre et représentent un atome d'hydrogène ou un groupe alcoyle en $C_1$ à $C_3$, un groupe représenté par la formule $OR^7$ où $R^7$ est un atome d'hydrogène ou un groupe alcoyle en $C_1$ à $C_4$, un groupe représenté par la formule:

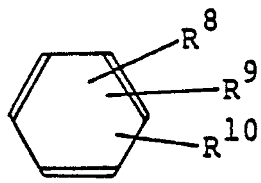
,

où $R^8$, $R^9$ et $R^{10}$ sont identiques ou différents l'un de l'autre et représentent un atome d'hydrogène ou un groupe alcoxy en $C_1$ à $C_3$, ou un groupe représenté par la formule:

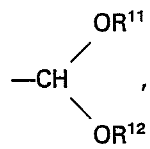
,

où $R^{11}$ et $R^{12}$ sont identiques ou différents l'un de l'autre et représentent un groupe alcoyle en $C_1$ à $C_3$; un groupe cycloalcoyle en $C_3$ à $C_8$, un groupe représenté par la formule:

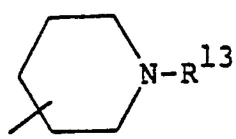
,

où $R^{13}$ est un atome d'hydrogène ou un groupe alcoyle en $C_1$ à $C_3$, ou un groupe représenté par la formule $-CH_2(CHOH)_4CH_2OH$; un groupe représenté par la formule:

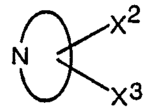
,

où

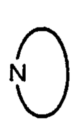

est un groupe amino cyclique à 3 à 10 chaînons ayant de 2 à 9 atomes de carbone, $X^2$ est un atome d'hydrogène ou un groupe alcoyle en $C_1$ à $C_4$ et $X^3$ est un atome d'hydrogène, un groupe alcoyle en $C_1$ à $C_3$, un groupe hydroxyle, un groupe représenté par la formule:

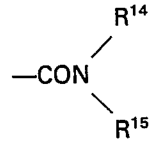 ,

où $R^{14}$ et $R^{15}$ sont identiques ou différents l'un de l'autre et représentent un atome d'hydrogène ou un groupe alcoyle en $C_1$ à $C_4$, un groupe hydroxyalcoyle en $C_1$ à $C_3$ ou un groupe amino cyclique ou non cyclique ayant de 2 à 6 atomes de carbone, ou $X^2$ et $X^3$ pris ensemble représentent un groupe =O ou le groupe de formule —$O(CH_2)_bO$—, où b vaut de 2 à 4, un groupe représenté par la formule

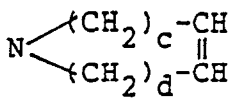 ,

où c et d sont identiques ou différents l'un de l'autre et valent de 1 à 4, un groupe représenté par la formule:

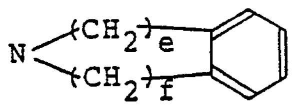 ,

où e et f sont identiques ou différents l'un de l'autre et valent de 1 à 4, ou un groupe représenté par la formule:

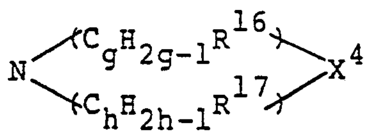

où g et h sont identiques ou différents l'un de l'autre et valent de 1 à 4, $R^{16}$ et $R^{17}$ sont identiques ou différents l'un de l'autre et représentent un atome d'hydrogène ou un groupe alcoyle en $C_1$ à $C_2$ et $X^4$ est un atome d'oxygène, un atome de soufre ou un groupe représenté par la formule $NR^{18}$ où $R^{18}$ est un atome d'hydrogène, un groupe alcoyle en $C_1$ à $C_3$, un groupe phényle représenté par la formule:

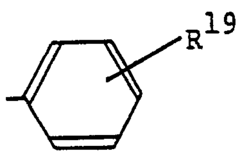

où $R^{19}$ est un atome d'hydrogène ou un groupe trifluorométhyle, ou un groupe représenté par la formule:

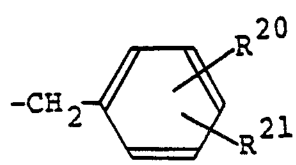

où $R^{20}$ et $R^{21}$ représentent des atomes d'hydrogène ou pris ensemble, représentent le groupe de formule: —$OCH_2O$—, ou ses sels.

2. Dérivé de rifamycine ou ses sels de la revendication 1, où $R^1$ est un groupe acétyle dans la formule générale (I).

3. Dérivé de rifamycine ou ses sels de la revendication 1, où $R^1$ est un atome d'hydrogène dans la formule général (I).

4. Dérivé de rifamycine ou ses sels de la revendication 1, 2 ou 3, où $R^2$ est un atome d'hydrogène dans la formule générale (I).

5. Dérivé de rifamycine ou ses sels des revendication 1, 2 ou 3, où $R^2$ est un groupe hydroxyle dans la formule générale (I).

6. Dérivé de rifamycine ou ses sels de la revendication 1, 2 ou 4, où $R^1$ est un groupe acétyle et $R^2$ un atome d'hydrogène dans la formule générale (I).

7. Dérivé de rifamycine ou ses sels de la revendication 1, 3 ou 4, où $R^1$ et $R^2$ représentent l'un et l'autre des atomes d'hydrogène dans la formule générale (I).

8. Dérivé de rifamycine ou ses sels de la revendication 1, 2, 3, 4, 5, 6 ou 7, où A est un groupe représenté par la formule

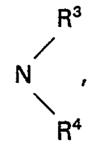 ,

où $R^3$ est un groupe alcoyle en $C_1$ à $C_5$ ou un groupe alcoxyalcoyle en $C_2$ à $C_6$ et $R^4$ est un groupe alcoyle en $C_1$ à $C_5$, un groupe représenté par la formule: $-\!\!(CH_2)_aX^1$, où a vaut 1 à 4 et $X^1$ est un groupe éthynyle, un groupe cyano, un groupe de formule:

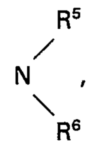 ,

où $R^5$ et $R^6$ sont identiques ou différents l'un de l'autre et représentent un atome d'hydrogène ou un groupe alcoyle en $C_1$ à $C_3$, un groupe représenté par la formule $OR^7$ où $R^7$ est un atome d'hydrogène ou un groupe alcoyle en $C_1$ à $C_4$, un groupe représenté par la formule:

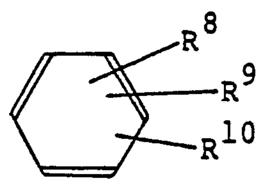 ,

où $R^8$, $R^9$ et $R^{10}$ sont identiques ou différents l'un de l'autre et représentent un atome d'hydrogène ou un groupe alcoxy en $C_1$ à $C_3$, ou un groupe représenté par la formule:

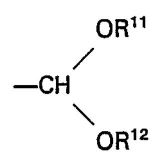

où $R^{11}$ et $R^{12}$ sont identiques ou différents l'un de l'autre et représentent un groupe alcoyle en $C_1$ à $C_3$; un groupe cycloalcoyle en $C_3$ à $C_8$, un groupe représenté par la formule:

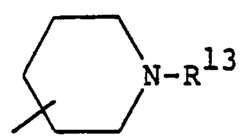

où $R^{13}$ est un atome d'hydrogène ou un groupe alcoyle en $C_1$ à $C_3$, ou un groupe représenté par la formule: $-CH_2(CHOH)_4CH_2OH$ dans la formule générale (I).

9. Dérivé de rifamycine ou ses sels de la revendication 1, 2, 3, 4, 5, 6 ou 7 où A est un groupe représenté par la formule:

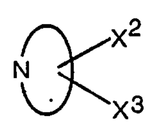 ,

où

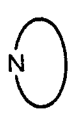

est un groupe amino cyclique à 3 à 10 chaînons avec de 2 à 9 atomes de carbone, $X^2$ est un atome d'hydrogène ou un groupe alcoyle en $C_1$ à $C_4$ et $X^3$ est un atome d'hydrogène, un groupe alcoyle en $C_1$ à $C_3$, un groupe hydroxyle, un groupe représénté par la formule:

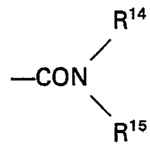 ,

où $R^{14}$ et $R^{15}$ sont indentiques ou différents l'un de l'autre et représentent un atome d'hydrogène ou un groupe alcoyle en $C_1$ à $C_4$, un groupe hydroxyalcoyle en $C_1$ à $C_3$ ou un groupe amino cyclique ou non cyclique ayant de 2 à 6 atomes de carbone, ou $X^2$ et $X^3$ pris ensemble représentent un groupe =O ou le groupe de formule $—O(CH_2)_bO—$, où b vaut de 2 à 4 dans la formule générale (I).

10. Dérivé de rifamycine ou ses sels de la revendication 1, 2, 3, 4, 5, 6 ou 7, où A est un groupe représenté par la formule:

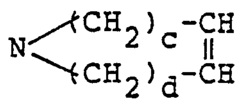 ,

où c et d sont identiques ou différents l'un de l'autre et valent de 1 à 4 ou un groupe représenté par la formule:

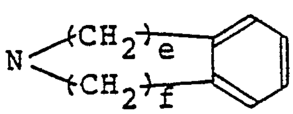 ,

où e et f sont identiques ou différents l'un de l'autre et valent de 1 à 4 dans la formule générale (I).

11. Dérivé de rifamycine ou ses sels de la revendication 1, 2, 3, 4, 5, 6 ou 7, où A est un groupe représenté par la formule:

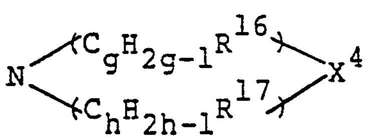 ,

où g et h sont identiques ou différents l'un de l'autre et valent de 1 à 4, $R^{16}$ et $R^{17}$ sont identiques ou différents l'un de l'autre et représentent un atome d'hydrogène ou un groupe alcoyle en $C_1$ à $C_2$ et $X^4$ est un atome d'oxygène, un atome de soufre ou un groupe représenté par la formule $NR^{18}$ où $R^{18}$ est un atome d'hydrogène, un groupe alcoyle en $C_1$ à $C_3$, un groupe phényle représenté par la formule:

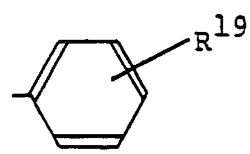 ,

où $R^{19}$ est un atome d'hydrogène ou un groupe trifluorométhyle, ou un groupe représenté par la formule:

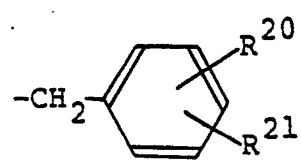 ,

où $R^{20}$ et $R^{21}$ représentent des atome d'hydrogène ou, lorsqu'ils sont ensemble, représentent le groupe de formule: $—OCH_2O—$ dans la formule générale (I).

12. Dérivé de rifamycine ou ses sels de la revendication 1, où $R^1$ est un groupe acétyle, $R^2$ est un atome d'hydrogène, $R^3$ est un groupe méthyle et $R^4$ est un groupe éthyle dans la formule générale (I).

13. Dérivé de rifamycine ou ses sels de la revendication 1, où $R^1$ est un groupe acétyle, $R^2$ est un atome d'hydrogène, et $R^3$ et $R^4$ sont tous les 2 des groupes éthyle dans la formule générale (I).

14. Dérivé de rifamycine ou ses sels de la revendication 1, où $R^1$ est un groupe acétyle, $R^2$ est un atome d'hydrogène et $R^3$ et $R^4$ sont tous les 2 des groupes propyle dans la formule générale (I).

15. Dérivé de rifamycine ou ses sels de la revendication 1, où $R^1$ est un groupe acétyle, $R^2$ est un atome d'hydrogène et $R^3$ comme $R^4$ sont des groupes éthoxyéthyle dans la formule générale (I).

16. Dérivé de rifamycine ou ses sels de la revendication 1, où $R^1$ est un groupe acétyle, $R^2$ est un atome d'hydrogène, $R^3$ est un groupe méthyle et $R^4$ est un groupe 2,2-diméthoxyéthyle dans la formule générale (I).

17. Dérivé de rifamycine ou ses sels de la revendication 1, ou $R^1$ est un groupe acétyle, $R^2$ est un atome d'hydrogène et A est un groupe représenté par la formule:

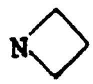

dans la formule générale (I).

18. Dérivé de rifamycine ou ses sels de la revendication 1, ou $R^1$ est un groupe acétyle, $R^2$ est un atome d'hydrogène et A est un groupe représenté par la formule:

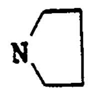

dans la formule générale (I).

19. Dérivé de rifamycine ou ses sels de la revendication 1, ou $R^1$ est un groupe acétyle, $R^2$ est un atome d'hydrogène et A est un groupe représenté par la formule:

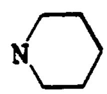

dans la formule générale (I).

20. Dérivé de rifamycine ou ses sels de la revendication 1, ou $R^1$ est un groupe acétyle, $R^2$ est un atome d'hydrogène et A est un groupe représenté par la formule:

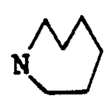

dans la formule générale (I).

21. Dérivé de rifamycine ou ses sels de la revendication 1, ou $R^1$ est un groupe acétyle, $R^2$ est un atome d'hydrogène et A est un groupe représenté par la formule:

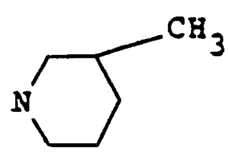

dans la formule générale (I).

22. Dérivé de rifamycine ou ses sels de la revendication 1, ou $R^1$ est un groupe acétyle, $R^2$ est un atome d'hydrogène et A est un groupe représenté par la formule:

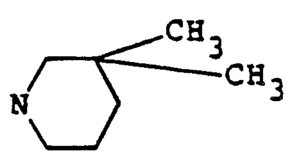

dans la formule générale (I).

23. Dérivé de rifamycine ou ses sels de la revendication 1, ou $R^1$ est un atome d'hydrogène, $R^2$ est un atome d'hydrogène et A est un groupe représenté par la formule:

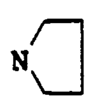 dans la formule générale (I).

24. Procédé de préparation d'un dérivé de rifamycine de formule générale (I)

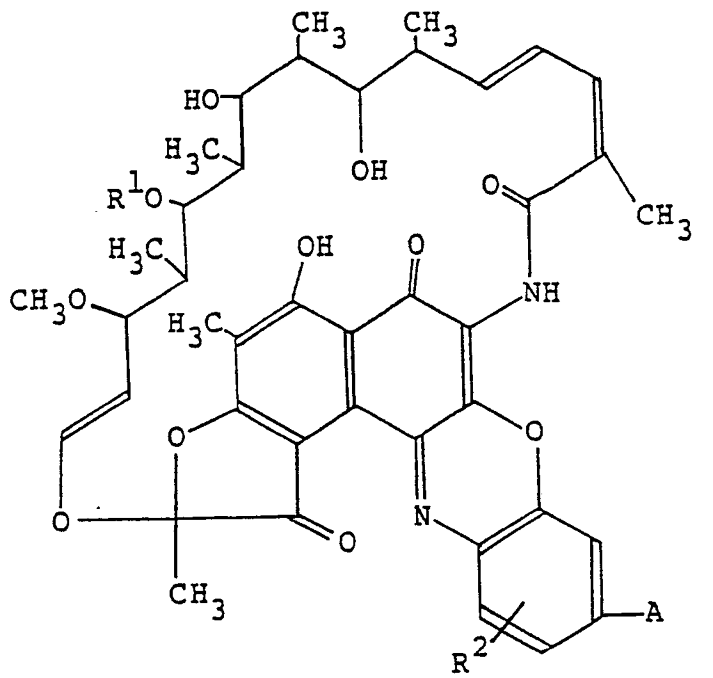

(I)

où $R^1$ est un atome d'hydrogène ou un groupe acétyle; $R^2$ est un atome d'hydrogène ou un groupe hydroxyle; A est tel que défini dans la revendication 1, dans lequel on fait réagir un dérivé de rifamycine de formule générale (II):

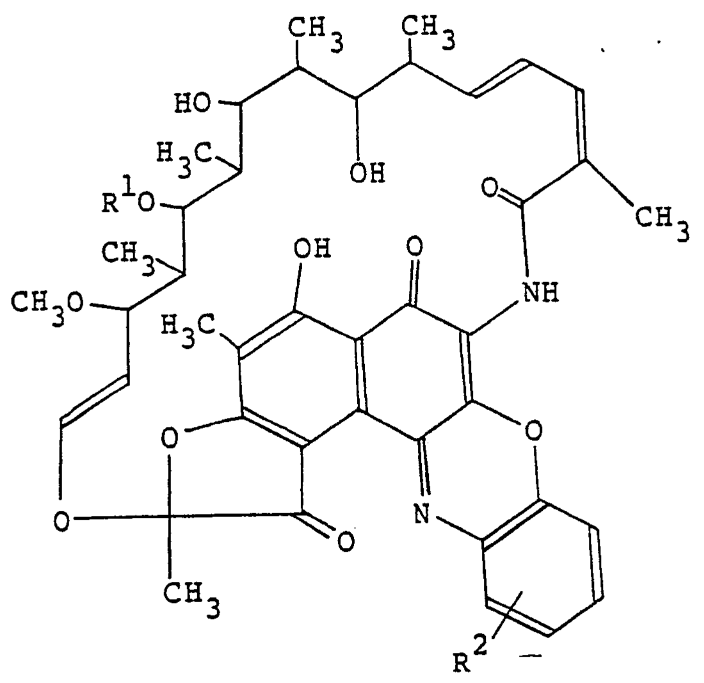

(II)

où $R^1$ et $R^2$ sont tels que définis ci-dessus, avec une amine de formule générale AH où A est tel que défini ci-dessus.

25. Procédé de la revendication 24, où la dérivé de rifamycine de formule générale (II) est mis à réagir avec l'amine de formule générale: AH en présence d'un agent oxydant.

26. Procédé de la revendication 24 ou 25, dans lequel l'agent oxydant est le dioxyde de manganèse.

27. Procédé de préparation d'un dérivé de rifamycine de formule générale (I) où $R^1$ est un atome d'hydrogène et $R^2$ et A sont tels que définis ci-dessus, dans lequel on hydrolyse un dérivé de rifamycine de

formule générale (I) où $R^1$ est un groupe acétyle, et $R^2$ et A sont tels que ci-dessus.

28. Procédé de la revendication 27, dans lequel l'agent employé pour l'hydrolyse est un hydroxyde de métal alcalin.

29. Agents antibactériens contenant un dérive de rifamycine de formule générale (I):

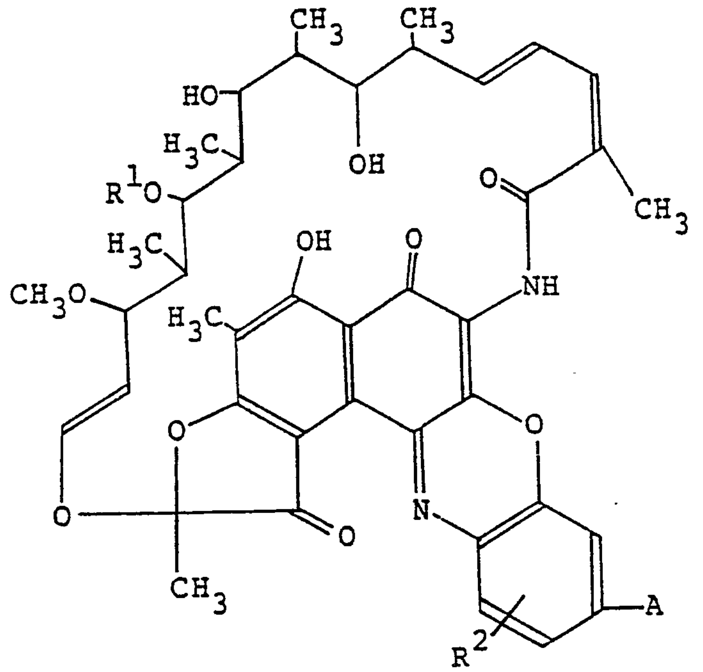

(I)

où $R^1$ est un atome d'hydrogène ou un groupe acétyle; $R^2$ est un atome d'hydrogène ou un groupe hydroxyle; A est tel que défini dans la revendication 1, ou leurs sels, comme composant efficace.